# EUROPÄISCHE PATENTSCHRIFT

(11) **EP 1 773 454 B1**
(45) Veröffentlichungstag und Bekanntmachung des Hinweises auf die Patenterteilung: **18.05.2011**
(21) Anmeldenummer: 05766699.2
(22) Anmeldetag: 08.07.2005
(51) Int. Cl.: A61Q 15/00, A61K 8/04

(54) **RÜCKSTANDSARMER DEODORANT-ODER ANTITRANSPIRANT-STIFT AUF BASIS EINER ÖL-IN-WASSER-DISPERSION**
LOW-RESIDUE DEODORANT OR ANTIPERSPIRANT STICK BASED ON AN OIL-IN-WATER DISPERSION
STICK DEODORANT OU ANTITRANSPIRANT SANS TRACES A BASE D'UNE DISPERSION HUILE DANS EAU

(30) Priorität: 28.07.2004 DE 102004036689
(43) Veröffentlichungstag der Anmeldung: 18.04.2007
(73) Patentinhaber: Henkel AG & Co. KGaA, 40589 Düsseldorf (DE)
(72) Erfinder: BANOWSKI, Bernhard, 40597 Düsseldorf (DE); WADLE, Armin, 40699 Erkrath (DE); CLAAS, Marcus, 40723 Hilden (DE)
(86) Internationale Anmeldenummer: PCT/EP2005/007414
(87) Internationale Veröffentlichungsnummer: WO 2006/012971

(56) Entgegenhaltungen:
- WO-A-02/083091
- DE-A1- 10 137 901
- DE-A1- 19 832 425
- US-A- 4 205 062
- VAUGHAN C D: "SOLUBILITY EFFECTS IN PRODUCT, PACKAGE, PENETRATION AND PRESERVATION" COSMETICS & TOILETRIES, WHEATON, IL, US, Bd. 103, Oktober 1988 (1988-10), Seiten 47-48,50,52,5, XP000872394 ISSN: 0361-4387 in der Anmeldung erwähnt
- VAUGHAN C.D.: 'USING SOLUBILITY PARAMETERS IN COSMETICS FORMULATION', 01 September 1985, JOURNAL OF THE SOCIETY COSMETIC CHEMISTS, SOCIETY OF COSMETIC CHEMISTS, US, PAGE(S) 319 - 333, ISSN 0037-9832 XP008009880
- GOHARSHADI E.K.; HESABI M.: 'Estimation of solubility parameters using equations of state' JOURNAL OF MOLECULAR LIQUIDS Bd. 113, 2004, Seiten 125 - 132

## Beschreibung

Die Erfindung betrifft Deodorant- oder Antitranspirant-Stifte auf Basis einer Öl-in-Wasser-Dispersion zur Applikation wasserlöslicher Wirkstoffe auf die Haut.

Handelsübliche Deodorantien und Antitranspirantien werden meistens als Sprays oder als Stift konfektioniert; daneben gibt es Roll-on-Präparate und Cremes im Markt. Viele stiftförmige Antitranspirant-Präparate werden als wasserfreie Suspensionsstifte formuliert. Derartige Präparate hinterlassen beim Anwender ein angenehm trockenes Hautgefühl nach dem Auftragen. Eine effektive Freisetzung der wasserlöslichen Antitranspirant-Wirkstoffe aus solchen Präparaten ist jedoch limitiert (vgl.: Chemistry and Technology of the Cosmetics and Toiletries Industry, Hrsg.: D. F. Williams und W. H. Schmitt, London: Blackie, 1996, 2. Auflage, S. 326), und es wird meist nicht das von vielen Verbrauchern geschätzte Frischegefühl erzielt. Die wasserfreien Präparate, insbesondere solche auf Basis von flüchtigen Siliconölen, haben den Nachteil, dass die dispergierten Wirkstoffe leicht zu sichtbaren Produktrückständen auf Haut und Kleidung führen. Außerdem sind solche Zubereitungen relativ kostspielig, da die Ölkomponenten als Wirkstoffträger teurer sind als Wasser. Unter der Druckbelastung bei der Applikation kommt es häufig zu einem Ausölen, was die kosmetische Akzeptanz dieser Präparate beim Anwender herabsetzt.

Gegenüber wasserfreien Stiften, wie sie zum Beispiel aus WO 94/24997 A1 und WO 00/67713 A1 bekannt sind, weisen Emulsionsstifte, wie sie zum Beispiel in WO 98/17238 A1, EP 281 288 A2, DE 2 335 549, US 4,725,431, EP 617 952 A1 und EP 291 334 offenbart sind, mehrere Vorteile auf. Der Ersatz der Wachs- und Ölzusätze durch Wasser macht die Emulsionsstifte kostengünstiger in der Herstellung. Die emulgierten Wachse vermitteln ein weiches, leichtes Hautgefühl, und schließlich können wasserlösliche kosmetische Wirkstoffe (also insbesondere auch Antitranspirant-Wirkstoffe) leichter an die Haut abgegeben werden, da sie in der wässrigen Phase der Emulsion bereits in gelöster Form vorliegen.

Die Emulsionsstifte des zitierten Standes der Technik sind auf Basis einer Wasser-in-Öl-Dispersion formuliert, die wasserlöslichen Wirkstoffe liegen also in der inneren, dispergierten Phase vor und müssen nach der Applikation erst durch die äußere, lipophile Schicht wandern, um ihren Wirkungsort auf der Haut zu erreichen. Damit weisen die bekannten Wasser-in-Öl-Emulsionsstifte hinsichtlich der Wirkstoffverfügbarkeit ähnliche Nachteile auf wie wasserfreie Suspensionsstifte.

DE 19749819 A1 offenbart wasser- und ölhaltige, wachsfreie Antitranspirant-Stifte auf Basis einer Öl-in-Wasser-Emulsion. Derartige Stifte weisen unzureichende kosmetische Eigenschaften auf, hinterlassen unangenehme klebrige und sichtbare Rückstände und zeigen eine für den längeren Gebrauch nicht ausreichende Stabilität. Ein Beispiel mit Glycerinmonostearat als W/O-Emulgator und Octyldodecanol als Ölkomponente weist eine mittelfeste Konsistenz und ein fettiges Hautgefühl auf und beginnt bereits bei 50 °C zu erweichen.

In der WO 99/59537 A1 sind wasserhaltige, ölfreie Deodorant-Stifte auf Basis einer (Lipid-oder-Öl)-in-Wasser-Emulsion offenbart, die Wachskomponenten mit einem Schmelzpunkt > 50°C, nichtionische Wasser-in-Öl-Emulgatoren, einen nichtionischen Öl-in-Wasser-Emulgator mit einem HLB-Wert von mehr als 7 und ein Polyol enthalten. Weiterhin sind in diesem Dokument mikroemulsionshaltige und PIT-emulsionshaltige Deodorant-Stifte offenbart, die Wachskomponenten mit einem Schmelzpunkt > 50°C, nichtionische Wasser-in-Öl-Emulgatoren, einen nichtionischen Öl-in-Wasser-Emulgator mit einem HLB-Wert von mehr als 7 und bei 20° C flüssige Öle enthalten. Derartige Stifte weisen ebenfalls unzureichende kosmetische Eigenschaften auf, hinterlassen unangenehme klebrige und sichtbare Rückstände und zeigen eine für den längeren Gebrauch nicht ausreichende Stabilität.

WO 02/083091 A1 offenbart strukturierte Antitranspirant-Zusammensetzungen in Form einer Wasser-in-Öl-Mikroemulsion. Der strukturelle Unterschied zwischen diesen Zusammensetzungen und den Öl-in-Wasser-Dispersionsstiften der vorliegenden Erfindung wird besonders deutlich durch den hohen Anteil an erfindungsgemäß ungünstigen Silicon- und Kohlenwasserstoffölen.

WO 02/017870 A2 offenbart wasserhaltige Antitranspirant-Stifte, die ein siliconiertes Polyamid als Konsistenzgeber bzw. Structurant enthalten.

WO 02/032914 A1 offenbart wasserhaltige Antitranspirant-Stifte, die acylierte Cellobiose als Konsistenzgeber bzw. Structurant sowie einen hohen Anteil an erfindungsgemäß ungünstigen Silicon- und Kohlenwasserstoffölen enthalten.

In den Offenlegungsschriften DE 199 62 878 A1 und DE 199 62 881 A1 sind Deodorant- oder Antitranspirant-Cremes auf Basis einer Öl-in-Wasser-Emulsion offenbart, die bei 21°C eine Viskosität von wenigstens 50000 mPa·s, bevorzugt im Bereich von 200000 - 1500000 mPa·s, aufweisen, das heißt, sie liegen in viskoser bis hochviskoser pastöser Form vor. Diese Cremes enthalten Wachskomponenten mit einem Schmelzpunkt > 50°C, nichtionische Wasser-in-Öl-Emulgatoren, nichtionische Öl-in-Wasser-Emulgator mit einem HLB-Wert von mehr als 7 und ein Polyol. Als weiche Cremes lassen sie sich entweder nur mit den Fingern applizieren, was von vielen Verbrauchern als unpraktisch abgelehnt wird, oder die Cremes müssen in spezielle Applikatoren abgefüllt werden, die deutlich kostspieliger sind als die Stifthülsen für die erfindungsgemäßen Deodorant- oder Antitranspirant-Stifte. Würde man die in DE 199 62 878 A1 und DE 199 62 881 A1 offenbarten Zusammensetzungen nach dem Erhitzen und Mischen statisch, das heißt ohne Rühren, abkühlen lassen, so würde man stiftähnliche Zusammensetzungen mit insgesamt ungünstigen Anwendungseigenschaften wie schlechter Haptik und/oder mangelnder Stabilität, zum Beispiel durch Phasenseparation oder Bildung von Kondenswasser, erhalten, da die Emulgatoren und die Öle nicht wie in der vorliegenden Erfindung aufeinander abgestimmt sind.

Es bestand daher die Aufgabe, eine Deodorant- oder Antitranspirant-Zusammensetzung zu entwickeln, die sich als effektiver Träger für wasserlösliche Wirkstoffe eignet und eine schnelle Freisetzung des Wirkstoffes auf der Haut ermöglicht.

Eine weitere Aufgabe- bestand darin, eine Deodorant- oder Antitranspirant-Zusammensetzung mit hervorragenden kosmetischen Pflegeeigenschaften zu entwickeln.

Eine weitere Aufgabe bestand darin, einen Deodorant- oder Antitranspirant-Stift zu entwickeln, der einerseits eine hohe Stabilität, das heißt Festigkeit, andererseits aber ein angenehmes Abgabeverhalten aufweist, also nicht zu fest ist, sondern sich leicht über die Haut streichen lässt und dabei eine ausreichende Produktmenge abgibt.

Eine weitere Aufgabe bestand darin, eine Deodorant- oder Antitranspirant-Zusammensetzung zu entwickeln, die beim Auftragen auf die Haut möglichst wenig klebrige oder sichtbare Rückstände hinterlässt.

Eine weitere Aufgabe bestand darin, eine Deodorant- oder Antitranspirant-Zusammensetzung zu entwickeln, die möglichst wenig sichtbare Rückstände auf der Kleidung, die mit der behandelten Haut in Kontakt kommt, hinterlässt.

Eine weitere Aufgabe bestand darin, eine Deodorant- oder Antitranspirant-Zusammensetzung zu entwickeln, die sich leicht von der Haut abwaschen lässt.

Eine weitere Aufgabe bestand darin, eine Deodorant- oder Antitranspirant-Zusammensetzung mit einem wirtschaftlich und anwendungstechnisch günstigen Kosten-LeistungsVerhältnis zu entwickeln.

Überraschend und für den Fachmann nicht vorhersehbar wurden diese Aufgaben gelöst durch den Gegenstand der vorliegenden Anmeldung, nämlich einen Deodorant- oder Antitranspirant-Stift in Form einer Öl-in-Wasser-Dispersion, enthaltend
a) insgesamt 4-20 Gew. -% mindestens einer Lipid- oder Wachskomponente mit einem Schmelzpunkt> 50°C, die nicht den Komponenten b) oder c) zuzurechnen ist,
b)ein nicht ionisches Öl-in-Wasser-Emulgatorgemisch mit einem mittleren HLB-Wert im Bereich von 10-19, enthaltend, bezogen auf die Gesamt Zusammensetzung, 0,5-10 Gew.-% mindestens eines nichtionischen Öl-in-Wasser-Emulgators mit einem HLB-Wert von mehr als 7,
c) insgesamt 0,1-15 Gew. -% mindestens eines nichtionischen Wasser-in-Öl-Emulgators mit einem HLB-Wert größer 1,0 und kleiner/gleich 7,0, der mit Wasser allein oder mit Wasser in Gegenwart eines hydrophilen Emulgators flüssigkristalline Strukturen ausbilden kann und der ausgewählt ist aus
   - linearen, gesättigten C₁₂ - C₃₀-Alkanolen,
   - Ethylenglycolmono- und diestern von linearen, gesättigten und ungesättigten C₁₂ - C₃₀-Carbonsäuren, die hydroxyliert sein können,
   - Glycerinmono- und diestern von linearen, gesättigten und ungesättigten C₁₂
   - C₃₀-Carbonsäuren, die hydroxyliert sein können,
   - Propylenglycolmono- und -diestern von linearen, gesättigten und ungesättig-ten C₁₂ - C₃₀-Carbonsäuren, die hydroxyliert sein können,
   - Sorbitanmono-, -di- und -triestern von linearen, gesättigten und ungesättigten C₁₂ - C₃₀-Carbon-säuren, die hydroxyliert sein können,
   - Pentaerythritylmono-, -di-, -tri- und -tetraestern von linearen, gesättigten und ungesättigten C₁₂ - C₃₀-Carbonsäuren, die hydroxyliert sein können,
   - Methylglucosemono- und -diestern von linearen, gesättigten und ungesättigten C₁₂ - C₃₀-Carbonsäuren, die hydroxyliert sein können,
   - Sterinen,
   - Alkanolen und Carbonsäuren mit jeweils 8-24 C-Atomen, insbesondere mit 16-22 C-Atomen, in der Alkylgruppe und 1 - 4 Ethylenoxid-Einheiten pro Molekül, die einen HLB-Wert größer 1,0 und kleiner/gleich 7,0 aufweisen,
   - Glycerinmonoethern gesättigter und/oder ungesättigter, verzweigter und/oder unverzweigter Alkohole einer Kettenlänge von 8 - 30, insbesondere 12-18 Kohlenstoffatomen,
   - Partialestern von Polyglycerinen mit n = 2 bis 10 Glycerineinheiten und mit 1 bis 5 gesättigten oder ungesättigten, linearen oder verzweigten, gegebenenfalls hydroxylierten C₈ - C₃₀-Fettsäureresten verestert, sofern sie einen HLB-Wert von kleiner/gleich 7 aufweisen,
   - sowie Mischungen der vorgenannten Substanzen,
      als Konsistenzgeber und/oder Wasserbinder,
d) insgesamt 3-20 Gew.-% mindestens eines bei 20 ° C flüssigen Öls das keine Duftstoffkomponente und kein etherisches Öl darstellt, wobei der (mittlere) Löslichkeitsparameter der Gesamtheit der enthaltenen Öle d) in Gegenwart von linearen gesättigten Fettalkoholen mit einer Kettenlänge von mindestens 8 Kohlenstoffatomen um maximal -2,93 (J/cm³)^{0,5} (-0,7 (cal/cm³)^{0,5}) bzw. maximal +2,93 (J/cm³)^{0,5} (+0,7 (cal/cm³)^{0,5}), und in Gegenwart von Wasser-in-Öl-Emulgatoren, die von linearen gesättigten Fettalkoholen mit einer Kettenlänge von mindestens 8 Kohlenstoffatomen verschieden sind, in Abwesenheit von linearen gesättigten Fettalkoholen mit einer Kettenlänge von mindestens 8 Kohlenstoffatomen um maximal -1,67 (J/cm³)^{0,5} (-0,4 (cal/cm³)^{0,5}) bzw. maximal +2,93 (J/cm³)^{0,5} (+0,7 (cal/cm³)^{0,5}) vom (mittleren) Löslichkeitsparameter des Wasser-in-Öl-Emulgators/der Wasser-in-Öl-Emulgatoren abweicht,
e) insgesamt 3-25 Gew.-% mindestens eines wasserlöslichen mehrwertigen C₂ - C₉-Alkanols mit 2-6 Hydroxyl-gruppen und/oder mindestens eines wasserlöslichen Polyethylenglycols mit 3-20 Ethylenoxid-Einheiten,
f) 10 bis weniger als 50 Gew.-% Wasser, bezogen auf die Gesamtzusammensetzung,
g) mindestens einen Deodorant- oder Antitranspirant-Wirkstoff,
   wobei der Stift
h) einen Penetrationskraftwert im Bereich von 150 - 800 Gramm-Kraft (g-force) bei einer Penetrationstiefe von 5,000 mm und
i) einen elektrischen Widerstand von maximal 300 kΩ (Kiloohm), gemessen nach dem in der Beschreibung offenbarten Verfahren, aufweist und
j) die Verfestigung des Deodorant- oder Antitranspirant-Stifts nicht auf der Basis von Seifengelen beziehungsweise Fettsäuresalz-Gelen und nicht auf der Basis von anorganischen und/oder organischen polymeren Hydrogelbildnern erfolgt, wobei unter Fettsäuren Alkan-, Alken- und Alkinsäuren mit mindestens 4 kohlenstoffatomen Verstanden werden, wobei der Löslichkeitsparameter nach einer Methode bestimmt wird, nach der Diisopropyladipat einen Löslichkeitsparameter von 35,42 (J/cm³)^{0,5} (8,46 (cal/cm³)^{0,5}) und Octyldodecanol einen Löslichkeitsparameter von 37,35 (J/cm³)^{0,5} (8,92 (cal/cm³)^{0,5}) aufweisen, wobei alle Mengenangaben auf die Gesamtzusammensetzung bezogen sind.

Die Lipid- oder Wachskomponente mit einem Schmelzpunkt > 50°C formt mit dem/den Öl/en und ggf. weiteren höherschmelzenden Lipid- oder Wachskomponenten eine Gelmatrix, die größere Mengen an Wasser und Polyol aufnehmen kann. Diese Strukturen, die durch gewisse Mengen an Wasser-in-Öl-Emulgatoren und Öl-in-Wasser-Emulgatoren stabilisiert sind, hinterlassen aufgrund ihres Wassergehaltes bei der Anwendung einen frischen, kühlenden Eindruck. Dabei werden die Emulgatoren so aufeinander abgestimmt, dass die erfindungsgemäßen Stiftzusammensetzungen in Form einer Öl-in-Wasser-Dispersion vorliegen. Ohne an diese Theorie gebunden sein zu wollen, wird vermutet, dass die Öl-in-Wasser-Emulgatoren zusammen mit einem Teil der Wasser-in-Öl-Emulgatoren lamellare Flüssigkristallphasen bilden, die mit einem Teil des Wassers zu einer hydrophilen Gelphase aufgebaut werden. Diese hydrophile Gelphase umgibt die wässrige Bulkphase. In dieser wässrigen Bulkphase wiederum sind die lipophilen Komponenten, umgeben von einer lipophilen Gelphase, die von den Wasser-in-Öl-Emulgatoren mit einem Teil der Öl-in-Wasser-Emulgatoren und etwas Wasser gebildet wird, dispergiert.

Der Antitranspirant-Wirkstoff ist in der äußeren, kontinuierlichen wässrigen Phase gelöst, so dass sich eine deutlich verbesserte und effizientere Wirkstoffabgabe im Vergleich zu den bekannten wasserfreien Suspensionsstiften und Wasser-in-Öl-Emulsionsstiften ergibt. Diese Wirkstofffreisetzung lässt sich indirekt sehr gut bestimmen über die Messung des elektrischen Widerstandes des jeweiligen Produktes. Die genaue Messanordnung und die Durchführung der Messung sind im folgenden beschrieben (siehe unten). Die erfindungsgemäßen Stifte weisen demnach einen elektrischen Widerstand von maximal 300 kΩ, bevorzugt von maximal 100 kΩ und besonders bevorzugt von maximal 80 kΩ auf. Die in WO 98/17238 A1 offenbarten Stifte hingegen weisen einen elektrischen Widerstand von mehr als 3000 kΩ auf.

Die Verfestigung der erfindungsgemäßen Deodorant- oder Antitranspirant-Stifte erfolgt nicht auf der Basis von Seifengelen beziehungsweise Fettsäuresalz-Gelen, wobei unter Fettsäuren Alkan-, Alken- und Alkinsäuren mit mindestens 4 Kohlenstoffatomen, die substituiert, beispielsweise mit Hydroxylgruppen, verstanden werden. In einer besonders bevorzugten Ausführungsform sind die erfindungsgemäßen Deodorant- oder Antitranspirant-Stifte frei von Seifengelen beziehungsweise Fettsäuresalz-Gelen, insbesondere frei von Lithium-, Natrium-, Kalium-, Ammonium-, Diethanolamin- und Triethanolamin-Salzen von Fettsäuren.

Die Verfestigung der erfindungsgemäßen Deodorant- oder Antitranspirant-Stifte erfolgt nicht auf der Basis von anorganischen und/oder organischen polymeren Hydrogelbildnern, wie Cellulosen, Cellulosederivaten, beispielsweise Hydroxyalkylcellulosen, Polyacrylaten, Veegum oder Bentone. In einer besonders bevorzugten Ausführungsform sind die erfindungsgemäßen Deodorant- oder Antitranspirant-Stifte frei von durch anorganische und/oder organische polymere Hydrogelbildner gebildeten Gelen.

Neben der günstigen Wirkstofffreisetzung bringt die Konfektionierung als Öl-in-Wasser-Dispersion weitere Vorteile mit sich. Zum einen kann die Zusammensetzung leicht von der Haut abgewaschen werden. Zum anderen bildet sich bei oder nach dem Auftragen auf die Haut zusammen mit der Hautfeuchtigkeit eine pflegende Öl-in-Wasser-Creme aus.

Überraschend und für den Fachmann nicht vorhersehbar wurde gefunden, dass die Ölkomponenten und der Wasser-in-Öl-Emulgator beziehungsweise das Wasser-in-Öl-Emulgatoren-Gemisch in Bezug auf ihre Löslichkeitsparameter aufeinander abgestimmt sein müssen, um Stiftzusammensetzungen mit anwendungstechnisch zufriedenstellenden Härten zu bilden. Bei der Definition des Löslichkeitsparameters im Sinne der vorliegenden Erfindung wird Bezug genommen auf die Veröffentlichung "Solubility - Effects in Product, Package, Penetration and Preservation" von Chr. D. Vaughan in Cosmetics & Toiletries, Vol. 103, October 1988, Seiten 47 - 69. Die dort veröffentlichten Werte der Löslichkeitsparameter sind in der Nicht-SI-Einheit (cal/cm³)^{0,5} notiert. Der Einfachheit halber soll in dieser Schrift diese Nicht-SI-Einheit beibehalten werden.

In den erfindungsgemäßen Stiftzusammensetzungen weichen der (mittlere) Löslichkeitsparameter der Gesamtheit der enthaltenen Öle d) in Gegenwart von linearen gesättigten Fettalkoholen mit einer Kettenlänge von mindestens 8 Kohlenstoffatomen um maximal -2,93 (J/cm³)^{0,5} (-0,7 (cal/cm³)^{0,5}) bzw. maximal +2,93 (J/cm³)^{0,} (+0,7 (cal/cm³)^{0,5}), bevorzugt um maximal -2,51 (J/cm³)^{0,5} (-0,6 (cal/cm³)^{0,5}) bzw. maximal +2,51 (J/cm³)^{0,5} (+0,6 (cal/cm³)^{0,5}), besonders bevorzugt um maximal -1,67 (J/cm³)^{0,5} (-0,4 (cal/cm³)^{0,5}) bzw. maximal +2,09 (J/cm³)^{0,5} (+0,5 (cal/cm³)^{0,5}), und in Gegenwart von Wasser-in-Öl-Emulgatoren, die von linearen gesättigten Fettalkoholen mit einer Kettenlänge von mindestens 8 Kohlenstoffatomen verschieden sind, in Abwesenheit von linearen gesättigten Fettalkoholen mit einer Kettenlänge von mindestens 8 Kohlenstoffatomen um maximal -1,67 (J/cm³)^{0,5} (-0,4 (cal/cm³)^{0,5}) bzw. maximal +2,93 (J/cm³)^{0,5} (+0,7 (callcm³)^{0,5}), bevorzugt um maximal -1,26 (J/cm³)^{0,5} (-0,3 (cal/cm³)^{0,5}) bzw. maximal +2,51 (J/cm³)^{0,5} (+0,6 (cal/cm³)^{0,5}), besonders bevorzugt um maximal -0,84 (J/cm³)^{0,5} (-0,2 (cal/cm³)^{0,5}) bzw. maximal +2,09 (J/cm³)^{0,5} (+0,5 (cal/cm³)^{0,5}), vom (mittleren) Löslichkeitsparameter des Wasser-in-Öl-Emulgators/der Wasser-in-Öl-Emulgatoren ab. Falls Wasser-in-Öl-Emulgatoren-Gemische oder Ölgemische eingesetzt werden, wird jeweils der mittlere Löslichkeitsparameter der Mischung betrachtet, wobei das arithmetische Mittel gemäß dem Gewichtsanteil der Einzelkomponenten betrachtet wird. Im Rahmen der Erfindung ist es weiterhin möglich, dass ein Gewichtsanteil der eingesetzten, bei 20 °C flüssigen Öle von bis zu 20 Gew.-% aus Ölen besteht, deren Löslichkeitsparameter um mehr als -1,67 (J/cm³)^{0,5} (-0,4 (cal/cm³)^{0,5}) bzw. -2,93 (J/cm³)^{0,5} (-0,7 (cal/cm³)^{0,5}) oder um mehr als +2,93 (J/cm³)^{0,5} (+0,7 (cal/cm³)^{0,5}) vom (mittleren) Löslichkeitsparameter des Wasser-in-Öl-Emulgator(gemisch)s abweicht. In einer besonders bevorzugten Ausführungsform der Erfindung sind keine bei 20° C flüssigen Öle enthalten, deren Löslichkeitsparameter um mehr als ± 4,19 (J/cm³)^{0,5} (± 1,0 (cal/cm³)^{0,5}), bevorzugt um ±2,93 (J/cm³)^{0,5} (±0,7 (cal/cm³)^{0,5}) und besonders bevorzugt um ±2,09 (J/cm³)^{0,5} (±0,5 (cal/cm³)^{,5}) vom (mittleren) Löslichkeitsparameter des Wasser-in-Öl-Emulgators/ der Wasser-in-Öl-Emulgatoren abweicht.

### Lipid- oder Wachsmatrix

Die Lipid- oder Wachsmatrix der erfindungsgemäßen Stiftzusammensetzungen umfasst insgesamt 4-20 Gew.-% bezogen auf die Gesamt Zussammensetzung, mindestens einer Lipid- oder Wachskomponente mit einem Schmelzpunkt > 50°C, die nicht den nichtionischen Öl-in-Wasser-Emulgatoren mit einem HLB-Wert von mehr als 7 oder den nichtionischen Wasser-in-Öl-Emulgatoren mit einem HLB-Wert größer 1,0 und kleiner/ gleich 7,0, die mit Wasser allein oder mit Wasser in Gegenwart eines hydrophilen Emulgators flüssigkristalline Strukturen ausbilden können, zuzurechnen ist.

Generell sind Wachse von fester bis brüchig harter Konsistenz, grob bis feinkristallin, durchscheinend bis opak, jedoch nicht glasartig, und schmelzen oberhalb von 50 °C ohne Zersetzung. Sie sind schon wenig oberhalb des Schmelzpunktes niedrigviskos und zeigen eine stark temperaturabhängige Konsistenz und Löslichkeit.

Erfindungsgemäß bevorzugt sind beispielsweise natürliche pflanzliche Wachse, z. B. Candelillawachs, Carnaubawachs, Japanwachs, Zuckerrohrwachs, Ouricourywachs, Korkwachs, Sonnenblumenwachs, Fruchtwachse wie Orangenwachse, Zitronenwachse, Grapefruitwachs, und tierische Wachse, z. B. Bienenwachs, Schellackwachs und Walrat. Im Sinne der Erfindung kann es besonders bevorzugt sein, hydrierte oder gehärtete Wachse einzusetzen. Als Wachskomponente sind auch chemisch modifizierte Wachse, insbesondere die Hartwachse, wie z. B. Montanesterwachse, hydrierte Jojobawachse und Sasolwachse, einsetzbar. Zu den synthetischen Wachsen, die ebenfalls erfindungsgemäß bevorzugt sind, zählen beispielsweise Polyalkylenwachse und Polyethylenglycolwachse, C₂₀-C₄₀-Dialkylester von Dimersäuren, C₃₀₋₅₀-Alkylbienenwachs sowie Alkyl- und Alkylarylester von Dimerfettsäuren.

Eine besonders bevorzugte Wachskomponente ist ausgewählt aus mindestens einem Ester aus einem gesättigten, einwertigen C₁₆-C₆₀-Alkohol und einer gesättigten C₈-C₃₆-Monocarbonsäure. Erfindungsgemäß zählen hierzu auch Lactide, die cyclischen Doppelester von α-Hydroxycarbonsäuren der entsprechenden Kettenlänge. Ester aus Fettsäuren und langkettigen Alkoholen haben sich für die erfindungsgemäße Zusammensetzung als besonders vorteilhaft erwiesen, weil sie der Antitranspirantzubereitung ausgezeichnete sensorische Eigenschaften und dem Stift insgesamt eine hohe Stabilität verleihen. Die Ester setzen sich aus gesättigten, verzweigten oder unverzweigten Monocarbonsäuren und gesättigten, verzweigten oder unverzweigten einwertigen Alkoholen zusammen. Auch Ester aus aromatischen Carbonsäuren bzw. Hydroxycarbonsäuren (z. B. 12-Hydroxystearinsäure) und gesättigten, verzweigten oder unverzweigten Alkoholen sind erfindungsgemäß einsetzbar, sofern die Wachskomponente einen Schmelzpunkt > 50°C hat. Besonders bevorzugt ist, die Wachskomponenten zu wählen aus der Gruppe der Ester aus gesättigten, verzweigten oder unverzweigten Alkancarbonsäuren einer Kettenlänge von 12 bis 24 C-Atomen und den gesättigten, verzweigten oder unverzweigten Alkoholen einer Kettenlänge von 16 bis 50 C-Atomen, die einen Schmelzpunkt > 50°C haben.

Insbesondere können als Wachskomponente C₁₆₋₃₆-Alkylstearate und C₁₈₋₃₈-Alkylhydroxystearoylstearate, C₂₀₋₄₀-Alkylerucate sowie Cetearylbehenat vorteilhaft sein. Das Wachs oder die Wachskomponenten weisen einen Schmelzpunkt > 50°C, bevorzugt > 60°C, auf.

Eine besonders bevorzugte Ausführungsform der Erfindung enthält als Wachskomponente ein C₂₀-C₄₀-Alkylstearat. Dieser Ester ist unter den Namen Kesterwachs^{®} K82H oder Kesterwachs^{®} K80H bekannt und wird von Koster Keunen Inc. vertrieben. Es handelt sich um die synthetische Nachahmung der Monoesterfraktion des Bienenwachses und zeichnet sich durch seine Härte, seine Ölgelierfähigkeit und seine breite Kompatibiltät mit Lipidkomponenten aus. Dieses Wachs kann als Stabilisator und Konsistenzregulator für W/O- und O/W-Emulsionen verwendet werden. Kesterwachs bietet den Vorteil, dass es auch bei geringen Konzentrationen eine exzellente Ölgelierfähigkeit aufweist und so die Stiftmasse nicht zu schwer macht und einen samtigen Abrieb ermöglicht. Eine weitere besonders bevorzugte Ausführungsform der Erfindung enthält als Wachskomponente Cetearylbehenat, d. h. Mischungen aus Cetylbehenat und Stearylbehenat. Dieser Ester ist unter dem Namen Kesterwachs^{®} K62 bekannt und wird von Koster Keunen Inc. vertrieben.

Weitere bevorzugte Lipid- oder Wachskomponenten mit einem Schmelzpunkt > 50°C sind die Triglyceride gesättigter und gegebenenfalls hydroxylierter C₁₂₋₃₀-Fettsäuren, wie gehärtete Triglyceridfette (hydriertes Palmöl, hydriertes Kokosöl, hydriertes Rizinusöl), Glyceryltribehenat (Tribehenin) oder Glyceryltri-12-hydroxystearat, weiterhin synthetische Vollester aus Fettsäuren und Glycolen oder Polyolen mit 2 - 6 Kohlenstoffatomen, solange sie einen Schmelzpunkt oberhalb von 50 °C aufweisen, beispielsweise bevorzugt C₁₈ - C₃₆ Acid Triglyceride (Syncrowax^{®} HGL-C).

Erfindungsgemäß ist als Wachskomponente hydriertes Rizinusöl, erhältlich z. B. als Handelsprodukt Cutina^{®} HR, besonders bevorzugt.

Weitere bevorzugte Lipid- oder Wachskomponenten mit einem Schmelzpunkt > 50°C sind die gesättigten linearen C₁₄ - C₃₆-Carbonsäuren, insbesondere Myristinsäure, Palmitinsäure, Stearinsäure und Behensäure sowie Mischungen dieser Verbindungen, z. B. Syncrowax^{®} AW 1C (C₁₈ - C₃₆-Fettsäuren) oder Cutina^{®} FS 45 (Palmitin- und Stearinsäure).

Bevorzugte erfindungsgemäße Deodorant- oder Antitranspirant-Stifte sind dadurch gekennzeichnet, dass die Lipid- oder Wachskomponente a) ausgewählt ist aus Estern aus einem gesättigten, einwertigen C₁₆-C₆₀-Alkanol und einer gesättigten C₈-C₃₆-Monocarbonsäure, insbesondere Cetylbehenat, Stearylbehenat und C₂₀-C₄₀-Alkylstearat, Glycerintriestern von gesättigten linearen C₁₂ - C₃₀-Carbonsäuren, die hydroxyliert sein können, Candelillawachs, Carnaubawachs, Bienenwachs, gesättigten linearen C₁₄ - C₃₆-Carbonsäuren sowie Mischungen der vorgenannten Substanzen.

Erfindungsgemäße Deodorant- oder Antitranspirant-Stifte sind dadurch gekennzeichnet, dass die Lipid- oder Wachskomponente/n a) insgesamt in Mengen von 4-20 Gew.-%, bevorzugt 8-15 Gew.-%, bezogen auf die Gesamtzusammensetzung, enthalten ist. In einer besonders bevorzugten Ausführungsform ist/sind der/die Ester aus einem gesättigten, einwertigen C₁₆-C₆₀-Alkohol und einer gesättigten C₈-C₃₈-Monocarbonsäure, der/die Lipid- oder Wachskomponente/n a) darstellt/darstellen, in Mengen von 2-10 Gew.-%, bevorzugt 2-6 Gew.-%, bezogen auf die Gesamtzusammensetzung, enthalten.

### Öl-in-Wasser-Emulgatoren

Die erfindungsgemäßen Stiftzusammensetzungen enthalten bezogen auf die Gesamtzusammensetzung, 0,5 - 10 Gew.-% mindestens eines nichtionischen Öl-in-Wasser-Emulgators mit einem HLB-Wert von mehr als 7. Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seite 913-916, aufgelistet sind. Für ethoxylierte Produkte wird der HLB-Wert nach der Formel HLB = (100 - L) : 5 berechnet, wobei L der Gewichtsanteil der lipophilen Gruppen, das heißt der Fettalkyl- oder Fettacylgruppen, in den Ethylenoxidaddukten, ausgedrückt in Gewichtsprozent, ist.

Bei der Auswahl erfindungsgemäß geeigneter nichtionischer Öl-in-Wasser-Emulgatoren ist es besonders bevorzugt, ein Gemisch von nichtionischen Öl-in-Wasser-Emulgatoren einzusetzen, um die Stabilität der erfindungsgemäßen Stiftzusammensetzungen optimal einstellen zu können. Die einzelnen Emulgatorkomponenten- liefern- dabei-einen-Anteilzum Gesamt-HLB-Wert oder mittleren HLB-Wert des Öl-in-Wasser-Emulgatorgemisches gemäß ihrem Mengenanteil an der Gesamtmenge der Öl-in-Wasser-Emulgatoren. Erfindungsgemäß beträgt der mittlere HLB-Wert des nichtionischen Öl-in-Wasser- Emulgatorgemisches 10-19, bevorzugt 12 - 18 und besonders bevorzugt 14-17. Um derartige mittlere HLB-Werte zu erzielen, werden bevorzugt Öl-in-Wasser-Emulgatoren aus den HLB-Wertbereichen 10 - 14, 14 - 16 und gegebenenfalls 16 - 19 miteinander kombiniert. Selbstverständlich können die Öl-in-Wasser-Emulgatorgemische auch nichtionische Emulgatoren, mit HLB-Werten im Bereich von > 7 - 10 und 19 - 20 enthalten; derartige Emulgatorgemische können erfindungsgemäß ebenfalls bevorzugt sein.

Bevorzugte erfindungsgemäße Deodorant- oder Antitranspirant-Stifte sind dadurch gekennzeichnet, dass die nichtionischen Öl-in-Wasser-Emulgatoren b) ausgewählt sind aus ethoxylierten C₈-C₂₄-Alkanolen mit durchschnittlich 10 - 100 Mol Ethylenoxid pro Mol, ethoxylierten C₈-C₂₄-Carbonsäuren mit durchschnittlich 10 - 100 Mol Ethylenoxid pro Mol, Silicon-Copolyolen mit Ethylenoxid-Einheiten oder mit Ethylenoxid- und Propylenoxid-Einheiten, Alkylmono- und -oligoglycosiden mit 8 bis 22 Kohlenstoffatomen im Alkylrest und deren ethoxylierten Analoga, ethoxylierten Sterinen, Partialestern von Polyglycerinen mit n = 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, sofern sie einen HLB-Wert von mehr als 7 aufweisen, sowie Mischungen der vorgenannten Substanzen.

Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 10 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet.

Die ethoxylierten C₈-C₂₄-Carbonsäuren haben die Formel R¹O(CH₂CH₂O)ₙH, wobei R¹O steht für einen linearen oder verzweigten gesättigten oder ungesättigten Acylrest mit 8 - 24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachyinsäure, Gadoleinsäure, Behensäure, Erucasäure und Brassidinsäure sowie deren technische Mischungen. Auch Addukte von 10 - 100 Mol Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat.

Besonders bevorzugt eingesetzt werden die C₁₂-C₁₈-Alkanole oder die C₁₂-C₁₈-Carbonsäuren mit jeweils 10 - 30 Einheiten Ethylenoxid pro Molekül sowie Mischungen dieser Substanzen, insbesondere Ceteth-12, Ceteth-20, Ceteth-30, Steareth-12, Steareth-20, Steareth-30, Laureth-12 und Beheneth-20.

Weiterhin werden vorzugsweiseC₈ - C₂₂-Alkylmono- und -oligoglycoside eingesetzt. C₈ - C₂₂-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare^{®} erhältlich sind, enthalten eine glucosidisch gebundene C₈-C₁₆-Alkylgruppe an einem Oligoglucosidrest, dessen mittlerer Oligomerisationsgrad bei 1 - 2 liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet.

Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Geeignete Handelsprodukte sind z. B. PEG-10 Soy Sterol, PEG-16 Soy Sterol und PEG-25 Soy Sterol.

Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, sofern sie einen HLB-Wert von mehr als 7 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexaglycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decagtycerintriisostearat, Decaglycerintristearat, Decaglycerintrioleat und Decaglycerintrihydroxystearat.

Erfindungsgemäße Deodorant- oder Antitranspirant-Stifte sind dadurch gekennzeichnet, dass der nichtionische Öl-in-Wasser-Emulgator b) in einer Gesamtmenge von 0,5 - 10 Gew.-%, besonders bevorzugt 1 - 4Gew.-% und außerordentlich bevorzugt 1,5 - 3 Gew.-%, bezogen auf die Gesamtzusammensetzung, enthalten ist.

### Wasser-in-Öl-Emulgatoren

Die erfindungsgemäßen Stiftzusammensetzungen enthalten weiterhin bezogen auf die Gesamtzusammensetzung, insgesamt 0,1-15 Gew.-% mindestens eines nichtionischen Wasser-in-Öl-Emulgators mit einem HLB-Wert größer 1,0 und kleiner/ gleich 7,0, der mit Wasser allein oder mit Wasser in Gegenwart eines hydrophilen Emulgators flüssigkristalline Strukturen ausbilden kann und der ausgewählt ist aus linearen, gesättigten C₁₂ - C₃₀-Alkanolen, Ethylenglycolmono- und diestern von linearen, gesättigten und ungesättigten C₁₂ - C₃₀-Carbonsäuren, die hydroxyliert sein können, Glycerinmono- und diestern von linearen, gesättigten und ungesättigten C₁₂ - C₃₀-Carbonsäuren, die hydroxyliert sein können, Propylenglycolmono- und -diestern von linearen, gesättigten und ungesättigten C₁₂ - C₃ₒ-Carbonsäuren, die hydroxyliert sein können, Sorbitanmono-, -di- und -triestern von linearen, gesättigten und ungesättigten C₁₂ - C₃₀-Carbonsäuren, die hydroxyliert sein können, Pentaerythritylmono-, -di-, -tri- und -tetraestern von linearen, gesättigten und ungesättigten C₁₂ C₃₀-Carbonsäuren, die hydroxyliert sein können, Methylglucosemono- und -diestern von linearen, gesättigten und ungesättigten C₁₂ - C₃₀-Carbonsäuren, die hydroxyliert sein können, Sterinen, Alkanolen und Carbonsäuren mit jeweils 8-24 C-Atomen, insbesondere mit 16 - 22 C-Atomen, in der Alkylgruppe und 1 - 4 Ethylenoxid-Einheiten pro Molekül, die einen HLB-Wert größer 1,0 und kleiner/gleich 7,0 aufweisen, Glycerinmonoethern gesättigter und/oder ungesättigter, verzweigter und/oder unverzweigter Alkohole einer Kettenlänge von 8 - 30, insbesondere 12 - 18 Kohlenstoffatomen, Partialestern von Polyglycerinen mit n = 2 bis 10 Glycerineinheiten und mit 1 bis 5 gesättigten oder ungesättigten, linearen oder verzweigten, gegebenenfalls hydroxylierten C₈ - C₃₀-Fettsäureresten verestert, sofern sie einen HLB-Wert von kleiner/gleich als 7 aufweisen, sowie Mischungen der vorgenannten Substanzen, als Konsistenzgeber und/oder Wasserbinder. Der/die Wasser-in-Öl-Emulgator/en trägt/tragen vor allem zum Aufbau der lipophilen Gelphase bei, die die dispergierte Lipid-/Wachs-/Ölphase umgibt, wie auch, wenn auch in geringerem Maße, zum Aufbau der hydrophilen Gelphase, die die wässrige Phase stabilisiert. Einige dieser Emulgatoren sind beispielsweise in Kirk-Othmer, "Encyclopedia of Chemical Technology", 3. Aufl., 1979, Band 8, Seite 913, aufgelistet. Für ethoxylierte Addukte lässt sich der HLB-Wert, wie bereits erwähnt, auch berechnen.

Erfindungsgemäß kann es bevorzugt sein, nur einen einzigen Wasser-in-Öl-Emulgator einzusetzen. In einer anderen bevorzugten Ausführungsform enthalten die erfindungsgemäßen Zusammensetzungen Mischungen, insbesondere technische Mischungen, von mindestens zwei Wasser-in-Öl-Emulgatoren. Unter einer technischen Mischung wird beispielsweise ein Handelsprodukt wie Cutina^{®} GMS verstanden, das eine Mischung aus Glycerylmonostearat und Glyceryldistearat darstellt.

Besonders vorteilhaft einsetzbare Wasser-in-Öl-Emulgatoren sind Stearylalkohol, Cetylalkohol, Glycerylmonostearat, insbesondere in Form der Handelsprodukte Cutina^{®} GMS und Cutina^{®} MD (ex Cognis), Glyceryldistearat, Glycerylmonocaprinat, Glycerylmonocaprylat, Glycerylmonolaurat, Glycerylmonomyristat, Glycerylmonopalmitat, Glycerylmonohydroxystearat, Glycerylmonooleat, Glycerylmonolanolat, Glyceryldimyristat, Glyceryldipalmitat, Glyceryldioleat, Propylenglycolmonostearat, Propylenglycolmonolaurat, Sorbitanmonocaprylat, Sorbitanmonolaurat, Sorbitanmonomyristat, Sorbitanmonopalmitat, Sorbitanmonostearat, Sorbitansesquistearat, Sorbitandistearat, Sorbitandioleat, Sorbitansesquioleat, Saccharosedistearat, Arachidylalkohol, Behenylalkohol, Polyethylenglycol(2)stearylether (Steareth-2), Steareth-5, Oleth-2, Diglycerinmonostearat, Diglycerinmonoisostearat, Diglycerinmonooleat, Diglycerindihydroxystearat, Diglycerindistearat, Diglycerindioleat, Triglycerindistearat, Tetraglycerinmonostearat, Tetraglycerindistearat, Tetraglycerintristearat, Decaglycerinpentastearat, Decaglycerinpentahydroxystearat, Decaglycerinpentaisostearat, Decaglycerinpentaoleat, Soy Sterol, PEG-1 Soy Sterol, PEG-5 Soy Sterol, PEG-2-monolaurat und PEG-2-monostearat.

Erfindungsgemäße Deodorant- oder Antitranspirant-Stifte sind dadurch gekennzeichnet, dass der/die ausgewählte/n nichtionische/n Wasser-in-Öl-Emulgator/en c) mit einem HLB-Wert größer 1,0 und kleiner/gleich 7,0, der/die mit Wasser allein oder mit Wasser in Gegenwart eines hydrophilen Emulgators flüssigkristalline Strukturen ausbilden kann/können, in einer Gesamtmenge von 0,1 - 15 Gew.-%, besonders bevorzugt 0,5 - 8 Gew.-% und außerordentlich bevorzugt 1 - 4 Gew.-%, bezogen auf das Gesamtgewicht der Zusammensetzung, enthalten ist/sind. Weiterhin können auch Mengen von 2 - 3 Gew.-%, bezogen auf das Gesamtgewicht der Zusammensetzung, erfindungsgemäß außerordentlich bevorzugt sein.

### Öle

Die erfindungsgemäßen Stiftzusammensetzungen enthalten weiterhin, bezogen auf die Gesamtzusammenszrung, insgesamt 3-20 Gew.-% mindestens eines bei 20 ° C flüssiges Öls das keine Duftstoffkomponente und kein etherisches Öl darstellt, wobei der (mittlere) Löslichkeitsparameter der Gesamtheit der enthaltenen Öle d) in Gegenwart von linearen gesättigten Fettalkoholen mit einer Kettenlänge von mindestens 8 Kohlenstoffatomen um maximal -2,93 (J/cm³)^{0,5} (-0,7 (cal/cm³)^{0,5}) bzw. maximal +2,93 (J/cm³)^{0,}. (+0,7 (cal/cm³)^{0,5}), bevorzugt um maximal -2,51 (J/cm³)^{0,5} (-0,6 (cal/cm³)^{0,5}) bzw. maximal +2,51 (J/cm³)^{0,5} (+0,6 (cal/cm³)^{0,5}), besonders bevorzugt um maximal -1,67 (J/cm³)^{0,5} (-0,4 (cal/cm³)^{0,5}) bzw. maximal +2,09 (J/cm³)^{0,5} (+0,5 (cal/cm³)^{0,5}), und in Gegenwart von Wasser-in-Öl-Emulgatoren, die von linearen gesättigten Fettalkoholen mit einer Kettenlänge von mindestens 8 Kohlenstoffatomen verschieden sind, in Abwesenheit von linearen gesättigten Fettalkoholen mit einer Kettenlänge von mindestens 8 Kohlenstoffatomen um maximal -1,67 (J/cm³)^{0,5} (-0,4 (cal/cm³)^{0,5}) bzw. maximal +2,93 (J/cm³)^{0,5} (+0,7 (cal/cm³)^{0,5}), bevorzugt um maximal -1,26 (J/cm³)^{0,5} (-0,3 (cal/cm³)^{0,5}) bzw. maximal +2,51 (J/cm³)^{0,5} (+0,6 (cal/cm³)^{0,5}), besonders bevorzugt um maximal -0,84 (J/cm³)^{0,5} (-0,2 (cal/cm³)^{0,5}) bzw. maximal +2,09 (J/cm³)^{0,5} (+0,5 (cal/cm³)^{0,5}), vom (mittleren) Löslichkeitsparameter des Wasser-in-Öl-Emulgators/der Wasser-in-Öl-Emulgatoren abweicht. Die Abstimmung des/der verwendeten Öls/Öle auf den/die verwendeten Wasser-in-Öl-Emulgator/en stellt einen wichtigen Parameter dieser Erfindung dar. Passen der Wasser-in-Öl-Emulgator und die Ölkomponente/n vom Löslichkeitsparameter her nicht innerhalb der beanspruchten Grenzen zusammen, erhält man Stifte mit einer anwendungstechnisch nicht befriedigenden Härte und Stabilität. Erfindungsgemäß bevorzugte Öle sind ausgewählt aus verzweigten gesättigten oder ungesättigten Fettalkoholen mit 6-30 Kohlenstoffatomen. Diese Alkohole werden häufig auch als Guerbet-Alkohole bezeichnet, da sie nach der Guerbet-Reaktion erhältlich sind. Bevorzugte Alkoholöle sind Hexyldecanol (Eutanol^{®} G 16, Guerbitol^{®} T 16), Octyldodecanol (Eutanol^{®} G, Guerbitol^{®} 20), 2-Ethylhexylalkohol und die Handelsprodukte Guerbitol^{®} 18, Isofol^{®} 12, Isofol^{®} 16, Isofol^{®} 24, Isofol^{®} 36, Isocarb^{®} 12, Isocarb^{®} 16 oder Isocarb^{®} 24.

Weitere bevorzugte Ölkomponenten sind Mischungen aus Guerbetalkoholen und Guerbetalkoholestern, z.B. das Handelsprodukt Cetiol^{®} PGL (Hexyldecanol und Hexyldecyllaurat).

Weitere erfindungsgemäß bevorzugte Öle sind ausgewählt aus den Triglyceriden von linearen oder verzweigten, gesättigten oder ungesättigten, gegebenenfalls hydroxylierten C₈-₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Olivenöl, Rapsöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol^{®} 318, Myritol^{®} 331 (Cognis) oder Miglyol^{®} 812 (Hüls) mit unverzweigten Fettsäureresten sowie Glyceryltriisostearin und die Handelsprodukte Estol^{®} GTEH 3609 (Uniqema) oder Myritol^{®} GTEH (Cognis) mit verzweigten Fettsäureresten.

Weitere erfindungsgemäß besonders bevorzugte Öle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte Öle sind ausgewählt aus den Anlagerungsprodukten von 1 bis 5 Propylenoxid-Einheiten an ein- oder mehrwertige C₈-₂₂-Alkanole wie Octanol, Decanol, Decandiol, Laurylalkohol, Myristylalkohol und Stearylalkohol, z. B. PPG-2-Myristylether und PPG-3-Myristylether (Witconol^{®} APM).

Für den Einsatz der im folgenden aufgezählten Öle in den erfindungsgemäßen Stiftzusammensetzungen ist zu beachten, dass ihr Anteil an der gesamten Ölmischung nur so groß ist, dass der mittlere Löslichkeitsparameter der gesamten Ölmischung, wie erfindungsgemäß beansprucht und vorstehend beschrieben, an den mittleren Löslichkeitsparameter des Wasser-in-Öl-Emulgators angepasst ist. Entsprechende Öle sind ausgewählt aus den Estern der linearen oder verzweigten gesättigten oder ungesättigten Fettalkohole mit 2-30 Kohlenstoffatomen mit linearen oder verzweigten gesättigten oder ungesättigten Fettsäuren mit 2-30 Kohlenstoffatomen, die hydroxyliert sein können. Dazu zählen Hexyldecylstearat (Eutanol^{®} G 16 S), Hexyldecyllaurat, Isodecylneopentanoat, Isononylisononanoat, 2-Ethylhexylpalmitat (Cegesoft^{®} C 24) und 2-Ethylhexylstearat (Cetiol^{®} 868). Ebenfalls bedingt geeignet sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und -dipalmitat.

Weitere Öle, die mit Rücksicht auf die Löslichkeitsparameter-Abstimmung nur in geringen Mengen oder gar nicht einsetzbar sind, sind ausgewählt aus den Anlagerungsprodukten von mindestens 6 Ethylenoxid- und/oder Propylenoxid-Einheiten an ein- oder mehrwertige C₃₋₂₂-Alkanole wie Butanol, Butandiol, Myristylalkohol und Stearylalkohol, z. B. PPG-14-Butylether (Ucon Fluid^{®} AP), PPG-9-Butylether (Breox^{®} B25), PPG-10-Butandiol (Macol^{®} 57) und PPG-15-Stearylether (Arlamol^{®} E).

Weitere Öle, die mit Rücksicht auf die Löslichkeitsparameter-Abstimmung nur in geringen Mengen oder gar nicht einsetzbar sind, sind ausgewählt aus den C₈-C₂₂-Fettalkoholestern einwertiger oder mehrwertiger C₂-C₇-Hydroxycarbonsäuren, insbesondere die Ester der Glycolsäure, Milchsäure, Äpfelsäure, Weinsäure, Citronensäure und Salicylsäure. Solche Ester auf Basis von linearen C_{14/15}-Alkanolen, z. B. C₁₂₋C₁₅-Alkyllactat, und von in 2-Position verzweigten C_{12/13}-Alkanolen sind unter dem Warenzeichen Cosmacol^{®} von der Firma Nordmann, Rassmann GmbH & Co, Hamburg, zu beziehen, insbesondere die Handelsprodukte Cosmacol^{®} ESI, Cosmacol^{®} EMI und Cosmacol^{®} ETI.

Weitere Öle, die mit Rücksicht auf die Löslichkeitsparameter-Abstimmung nur in geringen Mengen oder gar nicht einsetzbar sind, sind ausgewählt aus den symmetrischen, unsymmetrischen oder cyclischen Estern der Kohlensäure mit Fettalkoholen, z. B. Glycerincarbonat, Dicaprylylcarbonat (Cetiol^{®} CC) oder die Ester der DE 197 56 454 A1. Weitere Öle, die mit Rücksicht auf die Löslichkeitsparameter-Abstimmung nur in geringen Mengen oder gar nicht einsetzbar sind, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

Es kann erfindungsgemäß bevorzugt sein, Mischungen der vorgenannten Öle einzusetzen.

Bevorzugte erfindungsgemäße Deodorant- oder Antitranspirant-Stifte sind dadurch gekennzeichnet, dass das bei 20 °C flüssige Öl d) ausgewählt ist aus verzweigten gesättigten oder ungesättigten Fettalkoholen mit 6 - 30 Kohlenstoffatomen, Triglyceriden von linearen oder verzweigten, gesättigten oder ungesättigten, gegebenenfalls hydroxylierten C₈₋₃₀-Fettsäuren, Dicarbonsäureestern von linearen oder verzweigten C₂-C₁₀-Alkanolen, Estern von verzweigten gesättigten- oder ungesättigten Fettalkoholen- mit 2 - 30 Kohlenstoffatomen mit linearen oder verzweigten gesättigten oder ungesättigten Fettsäuren mit 2 - 30 Kohlenstoffatomen, die hydroxyliert sein können, Anlagerungsprodukten von 1 bis 5 Propylenoxid-Einheiten an ein- oder mehrwertige C₈₋₂₂-Alkanole, Anlagerungsprodukten von mindestens 6 Ethylenoxid und/oder Propylenoxid-Einheiten an ein- oder mehrwertige C₃₋₂₂-Alkanole, C₈₋C₂₂-Fettalkoholestern einwertiger oder mehrwertiger C₂-C₇-Hydroxycarbonsäuren, symmetrischen, unsymmetrischen oder cyclischen Estern der Kohlensäure mit Fettalkoholen, den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen, sowie Mischungen der vorgenannten Substanzen.

Erfindungsgemäße Deodorant- oder Antitranspirant-Stifte sind dadurch gekennzeichnet, dass das/die bei 20 °C flüssige/n Öl/e d) in einer Gesamtmenge von 3 - 20 Gew.-%, bevorzugt 5 - 14 Gew.-%, bezogen auf das Gesamtgewicht der Zusammensetzung, enthalten ist/sind.

In einer weiteren besonders bevorzugten Ausführungsform der Erfindung beträgt der Anteil an Öl/en, deren Löslichkeitsparameter um mehr als -1,67 (J/cm³)^{0,5} (-0,4 (cal/cm³)^{0,5}) bzw. -2,93 (J/cm³)^{0,5} (-0,7 (cal/cm³)^{0,5}) oder um mehr als +2,93 (J/cm³)^{0,5} (+0,7 (cal/cm³)^{0,5}) vom mittleren Wert der verwendeten Wasser-in-Öl-Emulgatoren abweicht, maximal 20 Gew.-%, bezogen auf das Gesamtgewicht an bei 20 ° C flüssigen Ölen. In einer weiteren besonders bevorzugten Ausführungsform der Erfindung sind keine bei 20 ° C flüssigen Öle enthalten, deren Löslichkeitsparameter um mehr als ±4,19 (J/cm³)^{0,5} (± 1,0 (cal/cm³)^{0,5}) vom (mittleren) Löslichkeitsparameter des Wasser-in-Öl-Emulgators/der Wasser-in-Öl-Emulgatoren c) abweicht.

Entsprechende weniger geeignete oder (je nach verwendetem Wasser-in-Öl-Emulgator) sogar ungeeignete Ölkomponenten sind zum Beispiel Siliconöle und Kohlenwasserstofföle.

Siliconöle, zu denen z. B. Dialkyl- und Alkylarylsiloxane, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen, weisen Löslichkeitsparameter im Bereich von etwa 23,9 (J/cm³)^{0,5} (5,7 (cal/cm³)^{0,5}) bis 26,4 (J/cm³)^{0,5} (6,3 (cal/cm³)^{0,5}) auf, was um mehr als 1,67 (J/cm³)^{0,5} (0,4 (cal/cm³)^{0,5}) vom Wert der meisten der erfindungsgemäß verwendeten Wasser-in-Öl-Emulgatoren abweicht.

Natürliche und synthetische Kohlenwasserstoffe wie beispielsweise Paraffinöle, Isohexadecan, Isoeicosan, Polyisobutene oder Polydecene, die beispielsweise unter der Bezeichnung Emery^{®} 3004, 3006, 3010 oder unter der Bezeichnung Ethylflo^{®} von Albemarle oder Nexbase^{®} 2004G von Nestle erhältlich sind, sowie 1,3-Di-(2-ethylhexyl)-cyclohexan (Cetiol^{®}S) gehören ebenfalls zu den erfindungsgemäß weniger bevorzugten Ölkomponenten.

Der Anteil der Siliconöle und/oder Kohlenwasserstoffe sollte daher in einer bevorzugten Ausführungsform der Erfindung nicht größer sein als 20 %, bezogen auf das Gesamtgewicht an bei 20°C flüssigen Ölen, ansonsten erreichen die erfindungsgemäßen Stifte nicht die anwendungstechnisch gewünschte Härte und Stabilität. In einer besonders bevorzugten Ausführungsform der Erfindung sind keine Siliconöle und/oder Kohlenwasserstoffe enthalten.

### Polyole

Die erfindungsgemäßen Stiftzusam mensetzungen enthalten weiterhin, bezogeng auf die Gesamtzusammensetzung, insgesamt 3-25 Gew.-% mindestens eines wasserlöslichen mehrwertigen C₂ - C₉-Alkanols mit 2 - 6 Hydroxylgruppen und/oder mindestens eines assertöstichen Polyethylenglycols mit 3 - 20 Ethylenoxid-Einheiten sowie Mischungen hiervon. Bevorzugt sind diese Komponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind. Auch Zucker und bestimmte Zuckerderivate wie Fructose, Glucose, Maltose, Maltitol, Mannit, Inosit, Sucrose, Trehalose und Xylose sind erfindungsgemäß geeignet.

Bevorzugte erfindungsgemäße Deodorant- oder Antitranspirant-Stifte sind dadurch gekennzeichnet, dass das mindestens eine wasserlösliche mehrwertige C₂ - C₉-Alkanol mit 2-6 Hydroxylgruppen und/oder mindestens eine wasserlösliche Polyethylenglycol mit 3 - 20 Ethylenoxid-Einheiten ausgewählt ist aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen.

Erfindungsgemäße Deodorant- oder Antitranspirant-Stifte sind dadurch gekennzeichnet, dass das mindestens eine wasserlösliche mehrwertige C₂ - C₉-Alkanol mit 2 - 6 Hydroxylgruppen und/oder mindestens eine wasserlösliche Polyethylenglycol mit 3 - 20 Ethylenoxid-Einheiten insgesamt in Mengen von 3 - 25 Gew.-%, bevorzugt 8 - 18 Gew.-%, bezogen auf die Gesamtzusammensetzung, enthalten ist.

### Wasser

Der Anteil des Wassers an der erfindungsgemäßen Zusammensetzung beträgt 10 bis weniger als 50 Gew.-%, bevorzugt 10 bis weniger als 30 Gew.-%, besonders bevorzugt 15 - 28 Gew.-%, außerordentlich bevorzugt 20 - 26 Gew.-%, jeweils bezogen auf die Gesamtzusammensetzung.

Die erfindungsgemäßen Stiftzusammensetzungen enthalten weiterhin mindestens einen Deodorant- und/oder Antitranspirant-Wirkstoff.

### Deodorant-Wirkstoffe

Erfindungsgemäß bevorzugte Deodorant-Wirkstoffe sind Geruchsabsorber, desodorierend wirkende Ionenaustauscher, keimhemmende Mittel, präbiotisch wirksame Komponenten sowie Enzyminhibitoren oder, besonders bevorzugt, Kombinationen der genannten Wirkstoffe.

Silicate dienen als Geruchsabsorber, die auch gleichzeitig die rheologischen Eigenschaften der erfindungsgemäßen Zusammensetzung vorteilhaft unterstützen. Zu den erfindungsgemäß besonders vorteilhaften Silicaten zählen vor allem Schichtsilicate und unter diesen insbesondere Montmorillonit, Kaolinit, Ilit, Beidellit, Nontronit, Saponit, Hectorit, Bentonit, Smectit und Talkum. Weitere vorteilhafte Geruchsabsorber sind beispielsweise Zeolithe, Zinkricinoleat, Cyclodextrine, bestimmte Metalloxide, wie z. B. Aluminiumoxid, sowie Chlorophyll. Sie werden bevorzugt in einer Menge von 0,1 - 10 Gew.-%, besonders bevorzugt 0,5 - 7 Gew.-% und außerordentlich bevorzugt 1 - 5 Gew.-%, jeweils bezogen auf die Gesamtzusammensetzung, eingesetzt.

Unter keimhemmenden oder antimikrobiellen Wirkstoffen werden erfindungsgemäß solche Wirkstoffe verstanden, die die Zahl der an der Geruchsbildung beteiligten Hautkeime reduzieren bzw. deren Wachstum hemmen. Zu diesen Keimen zählen unter anderem verschiedene Spezies aus der Gruppe der Staphylokokken, der Gruppe der Corynebakterien, Anaerokokken und Mikrokokken.

Als keimhemmende oder antimikrobielle Wirkstoffe erfindungsgemäß bevorzugt sind insbesondere Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzedoniumchlorid. Weiterhin sind Phenol, Phenoxyethanol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, α-Monoalkylglycerinether mit einem verzweigten oder linearen gesättigten oder ungesättigten, gegebenenfalls hydroxylierten C₆ - C₂₂-Alkylrest, besonders bevorzugt α-(2-Ethylhexyl)glycerinether, im Handel erhältlich als Sensiva^{®} SC 50 (ex Schülke & Mayr), Carbonsäureester des Mono-, Di- und Triglycerins (z. B. Glycerinmonolaurat, Diglycerinmonocaprinat), Lantibiotika sowie Pflanzenextrakte (z. B. grüner Tee und Bestandteile des Lindenblütenöls) einsetzbar.

Weitere bevorzugte Deodorant-Wirkstoffe sind ausgewählt aus sogenannten präbiotisch wirksamen Komponenten, worunter erfindungsgemäß solche Komponenten zu verstehen sind, die nur oder zumindest überwiegend die geruchsbildenden Keime der Hautmikroflora hemmen, nicht aber die erwünschten, das heißt, die nicht-geruchsbildenden Keime, die zu einer gesunden Hautmikroflora gehören. Explizit sind hier die Wirkstoffe, die in den Offenlegungsschriften DE 10333245 und DE 10 2004 011 968 als präbiotisch wirksam offenbart sind, mit einbezogen; dazu gehören Nadelbaumextrakte, insbesondere aus der Gruppe der Pinaceae, und Pflanzenextrakte aus der Gruppe der Saplndaceae, Araliaceae, Lamiaceae und Saxifragaceae, insbesondere Extrakte aus *Picea spp., Paullinia sp., Panax sp., Lamium album* oder *Ribes nigrum* sowie Mischungen dieser Substanzen.

Weitere bevorzugte Deodorant-Wirkstoffe sind ausgewählt aus den keimhemmend wirkenden Parfümölen und den Deosafe-Parfümölen, die von der Firma Symrise, vormals Haarmann und Reimer, erhältlich sind.

Zu den Enzyminhibitoren gehören Stoffe, die die für die Schweißzersetzung verantwortlichen Enzyme, insbesondere die Arylsulfatase, β-Giucuronidase, Aminoacylase, Esterasen, Lipasen und/oder Lipoxigenase, hemmen, z. B. Trialkylcitronensäureester, insbesondere Triethylcitrat, oder Zinkglycinat.

Bevorzugle erfindungsgemäße Deodorant- oder Antitranspirant-Stifte sind dadurch gekennzeichnet, dass der mindestens eine Deodorant-Wirkstoff ausgewählt ist aus Arylsulfatase-Inhibitoren, β-Glucuronldase-Inhibltoren, Aminoacylase-Inhibitoren, Esterase-Inhibitoren, Lipase-Inhibitoren und Lipoxigenase-Inhibitoren, α-Monoalkylglycerinethern mit einem verzweigten oder linearen gesättigten oder ungesättigten, gegebenenfalls hydroxylierten C₆ - C₂₂ Alkylrest, insbesondere α-(2-Ethythexyl)glycerinether, Phenoxyethanol, keimhemmend wirkenden Parfümölen, Deosafe-Parfümölen, präbiotisch wirksamen Komponenten, Trialkylcitronensäureestern, insbesondere Triethylcitrat, Wirkstoffen, die die Zahl der an der Geruchsbildung beteiligten Hautkeime aus der Gruppe der Staphylokokken, Corynebakterien, Anaerokokken und Mikrokokken reduzieren bzw. deren Wachstum hemmen, Zinkverbindungen, insbesondere Zinkphenolsulfonat und Zinkricinoleat, Organohalogenverbindungen, insbesondere Triclosan, Chlorhexidin, Chlorhexidingluconat und Benzalkoniumhalogeniden, quartären Ammoniumverbindungen, insbesondere Cetylpyridiniumchlorid, Geruchsabsorbern, insbesondere Silikaten und Zeolithen, Natriumbicarbonat, Lantibiotika, sowie Mischungen der vorgenannten Substanzen.

Bevorzugte erfindungsgemäße Deodorant- oder Antitranspirant-Stifte sind dadurch gekennzeichnet, dass der mindestens eine Deodorant-Wirkstoff in einer Gesamtmenge von 0,1 - 10 Gew.%, bevorzugt 0,2 - 7 Gew.%, besonders bevorzugt 0,3 - 5 Gew.-% und außerordentlich bevorzugt 0,4 - 1,0 Gew.%, bezogen auf das Gesamtgewicht der Aktivsubstanz in der Gesamtzusammensetzung, enthalten ist.

### Antitranspirant-Wirkstoffe

Bevorzugte erfindungsgemäße Deodorant- oder Antitranspirant-Stifte sind dadurch gekennzeichnet, dass mindestens ein Antitranspirant-Wirkstoff, ausgewählt aus den wasserlöslichen adstringierenden anorganischen und organischen Salzen des Aluminiums, Zirkoniums und Zinks bzw. beliebigen Mischungen dieser Salze, enthalten ist. Besonders bevorzugte Antitranspirant-Wirkstoffe sind ausgewählt aus den Aluminiumchlorhydraten, insbesondere den Aluminiumchlorhydraten mit der allgemeinen Formel [Al₂(OH)₅Cl · 2-3 H₂O]ₙ, die in nicht-aktivierter oder in aktivierter (depolymerisierter) Form vorliegen können, weiterhin Aluminiumsesquichlorhydrat, Aluminiumchlorhydrex-Propylenglykol (PG) oder -Polyethylenglykol (PEG), Aluminiumsesquichlorhydrex-PG oder -PEG, Aluminium-PG-dichlorhydrex oder Aluminium-PEGdichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirkonium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat (KAI(SO₄)₂ - 12 H₂O, Alaun), Aluminiumundecylenoylkollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Komplexe von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat und Zirkoniumchlorohydrat. Erfindungsgemäß wird unter Wasserlöslichkeit eine Löslichkeit von wenigstens 5 Gew.-% bei 20 °C verstanden, das heißt, dass Mengen von wenigstens 5 g des Antitranspirant-Wirkstoffes in 95 g Wasser bei 20 °C löslich sind. Die Antitranspirant-Wirkstoffe können als wässrige Lösungen eingesetzt werden.

Besonders bevorzugte erfindungsgemäße Deodorant- oder Antitranspirant-Stifte sind dadurch gekennzeichnet, dass der mindestens eine Antitranspirant-Wirkstoff in einer Menge von 3 - 25 Gew.-%, vorzugsweise 5 - 22 Gew.-% und insbesondere 10 - 20 Gew.%, enthalten ist, bezogen auf das Gesamtgewicht der Aktivsubstanz in der Gesamtzusammensetzung. In einer besonders bevorzugten Ausführungsform enthält die Zusammensetzung ein adstringierendes Aluminiumsalz, insbesondere Aluminiumchlorohydrat, das beispielsweise pulverförmig als Micro Dry^{®} Ultrafine von Reheis, in Form einer wässrigen Lösung als Locron^{®} L von Clariant, als Chlorhydrol^{®} sowie in aktivierter Form als Reach^{®} 501 von Reheis vertrieben wird. Unter der Bezeichnung Reach^{®} 301 wird ein Aluminiumsesquichlorohydrat von Reheis angeboten, das ebenfalls besonders bevorzugt ist. Auch die Verwendung von Aluminium-Zirkonium-Tetrachlorohydrex-Glycin-Komplexen, die beispielsweise von Reheis unter der Bezeichnung Rezal^{®} 36G im Handel sind, kann erfindungsgemäß besonders bevorzugt sein.

Die erfindungsgemäßen Stiftzusammensetzungen können in einer weiteren besonders bevorzugten Ausführungsform sowohl mindestens einen Deodorant- als auch mindestens einen Antitranspirant-Wirkstoff enthalten.

### Niedrigschmelzende Lipid- oder Wachskomponente

Besonders bevorzugte erfindungsgemäße Deodorant- oder Antitranspirant-Stifte sind dadurch gekennzeichnet, dass mindestens eine Lipid- oder Wachskomponente mit einem Schmelzpunkt im Bereich von 25 - < 50°C, ausgewählt aus Kokosfettsäureglycerinmono-, -di- und -triestern, Butyrospermum Parkii (Shea Butter) und Estern von gesättigten, einwertigen C₈-C₁₆-Alkoholen mit gesättigten C₁₂-C₁₈-Monocarbonsäuren sowie Mischungen dieser Substanzen, enthalten ist. Diese niedriger schmelzenden Lipid- oder Wachskomponenten ermöglichen eine Konsistenzoptimierung des Produktes und eine Minimierung der sichtbaren Rückstände auf der Haut. Besonders bevorzugt sind Handelsprodukte mit der INCI-Bezeichnung Cocoglycerides, insbesondere die Handelsprodukte Novata^{®} (ex Cognis), besonders bevorzugt Novata^{®} AB, ein Gemisch aus C₁₂-C₁₈-Mono-, Di- und Triglyceriden, das im Bereich von 30 - 32°C schmilzt, sowie die Produkte der Softisan-Reihe (Sasol Germany GmbH) mit der INCI-Bezeichnung Hydrogenated Cocoglycerides, insbesondere Softisan 100, 133, 134, 138, 142. Weitere bevorzugte Ester von gesättigten, einwertigen C₁₂C₁₈-Alkoholen mit gesättigten C₁₂-C₁₈-Monocarbonsäuren sind Stearyllaurat, Cetearylstearat (z. B. Crodamol^{®} CSS), Cetylpalmitat (z. B. Cutina^{®} CP) und Myristylmyristat (z. B. Cetiol^{®} MM).

Weitere besonders bevorzugte erfindungsgemäße Deodorant- oder Antitranspirant-Stifte sind dadurch gekennzeichnet, dass die mindestens eine Lipid- oder Wachskomponente mit einem Schmelzpunkt im Bereich von 25 - < 50°C in Mengen von 0,01 bis 20 Gew.-%, bevorzugt 3 - 20 Gew.%, besonders bevorzugt 5 - 18 Gew.% und außerordentlich bevorzugt 6 -15 Gew.%, bezogen auf die Gesamtzusammensetzung, enthalten ist.

### Füllstoffe

Besonders bevorzugte erfindungsgemäae Deodorant- oder Antitranspirant-Stifte sind dadurch gekennzeichnet, dass sie zur Verbesserung der Stiftkonsistenz und der sensorischen Eigenschaften weiterhin mindestens einen festen, wasserunlöslichen teilchenförmigen Füllstoff enthalten. In einer außerordentlich bevorzugten Ausführungsform ist dieser Füllstoff ausgewählt aus gegebenenfalls modifizierten Stärken (z. B aus Mais, Reis, Kartoffeln) und Stärkederivaten, die gewünschtenfalls vorverkleistert sind, insbesondere Stärkederivaten vom Typ DRY FLO^{®}, Cellulose und Cellulosederivaten, Siliciumdioxid, Kieselsäuren, z. B. Aerosil^{®}-Typen, sphärischen Polyalkylsesquisiloxan-Partikeln (insbesondere Aerosil^{®} R972 und Aerosil^{®} 200V von Degussa), Kieselgelen, Talkum, Kaolin, Tonen, z. B. Bentoniten, Magnesiumaluminiumsilikaten, Bornitrid, Lactoglobulinderivaten, z. B. Natrium-C₈₋₁₆-Isoalkylsuccinyllactoglobulinsulfonat, von Brooks Industries erhältlich als Handelsprodukt Biopol^{®} OE, Glaspulvern, Polymerpulvern, insbesondere aus Polyolefinen, Polycarbonaten, Polyurethanen, Polyamiden, z. B. Nylon, Polyestern, Polystyrolen, Polyacrylate, (Meth)acrylat- oder (Meth)acrylat-Vinyliden-Copolymeren, die vernetzt sein können, oder Siliconen, sowie Mischungen dieser Substanzen. Polymerpulver auf Basis eines Polymethacrylat-Copolymers sind z. B. als Handelsprodukt Polytrap^{®} 6603 (Dow Corning) erhältlich. Andere Polymerpulver, z. B. auf Basis von Polyamiden, sind unter der Bezeichnung Orgasol^{®} 1002 (Polyamid-6) und Orgasol^{®} 2002 (Polyamid-12) von Elf Atochem erhältlich. Weitere Polymerpulver, die sich für den erfindungsgemäßen Zweck eignen, sind z. B. Polymethacrylate (Micropearl^{®} M von SEPPIC oder Plastic Powder A von NIKKOL), Styrol-Divinylbenzol-Copolymeren (Plastlc Powder FP von NIKKOL), Polyethylen- und Polypropylen-Pulver (ACCUREL^{®} EP 400 von AKZO) oder auch Siliconpolymere (Silicone Powder X2-1605 von Dow Corning). Besonders bevorzugte erfindungsgemäße Deodorant- oder Antitranspirant-Stifte sind dadurch gekennzeichnet, dass sie mindestens einen festen, wasserunlöslichen teilchenförmigen Füllstoff in einer Gesamtmenge von 0,01 bis 20 Gew.-%, bevorzugt 5 - 15 Gew.-%, jeweils bezogen auf die Gesamtzusammensetzung, enthalten.

### Duftstoffe

Besonders bevorzugte erfindungsgemäße Deodorant- oder Antitranspirant-Stifte sind dadurch gekennzeichnet, dass weiterhin mindestens eine Duftstoffkomponente enthalten ist.

Als Duftstoffkomponente können Parfüme, Parfümöle oder Parfümölbestandteile eingesetzt werden. Parfümöle bzw. Duftstoffe können erfindungsgemäß einzelne RiechstoffVerbindungen. z. B. die synthetischen Produkte vom Typ der Ester, Ether, Aldehyde, Ketone, Alkohole und Kohlenwasserstoffe sein. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Zu den Ethern zählen beispielsweise Benzylethylether und Ambroxan , zu den Aldehyden z.B. die linearen Alkanale mit 8 - 18 C-Atomen, Citral, Citronellal, Citronellyloxy-acetaldehyd, Cyclamenaldehyd, Lilial und Bourgeonal, zu den Ketonen z.B. die Jonone, alpha-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnoie erzeugen.

Solche Parfümöle können auch natürliche Riechstoffgemische enthalten, wie sie aus pflanzlichen Quellen zugänglich sind, z.B. Pine-, Citrus-, Jasmin-, Patchouly-, Rosen-oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelhofzöl.

Um wahrnehmbar zu sein, muss ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrücke in "Kopfnote" (top note), Herz- bzw. Mittelnote" (middle note bzw. body) sowie "Basisnote" (end note bzw. dry out) unterteilt. Da die Geruchswahrnehmung zu einem großen Teil auch auf der Geruchsintensität beruht, besteht die Kopfnote eines Parfüms bzw. Duftstoffs nicht allein aus leichtflüchtigen Verbindungen, während die Basisnote zum größten Teil aus weniger flüchtigen, d.h. haftfesten Riechstoffen besteht. Bei der Komposition von Parfüms konnen leichter flüchtige Riechstoffe beispielsweise an bestimmte Fixative gebunden werden, wodurch ihr zu schnelles Verdampfen verhindert wird. Bei der nachfolgenden Einteilung der Riechstoffe in leichter flüchtige" bzw. haftfeste" Riechstoffe ist also über den Geruchseindruck und darüber, ob der entsprechende Riechstoff als Kopf- oder Herznote wahrgenommen wird, nichts ausgesagt.

Haftfeste Riechstoffe, die im Rahmen der vorliegenden Erfindung einsetzbar sind, sind beispielsweise die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Resmarinöl, Sendelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl.

Aber auch die höhersiedenden bzw. festen Riechstoffe natürlichen oder synthetischen Ursprungs können im Rahmen der vorliegenden Erfindung als haftfeste Riechstoffe bzw, Riechstoffgemische, also Duftstoffe, eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Mathyl-n-nonylacetaldehyd, Methyl-n-nonylketon, Muskon, β-Naphthotethylether, β-Naphtholmethylether, Nerol, Nitrobenzol, n-Nonylaldehyd, Nonylakohol, n-Octylaldehyd, p-Oxy-Acetophenon. Pentadekanolid, β-Phenylethylakohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimatalkohol, Zimtsäure, Zimtsäureethylester, Zimtsäurebenzylester.

Zu den leichter flüchtigen Riechstoffen zählen insbesondere die niedriger siedenden Riechstoffe natürlichen oder synthetischen Usprung, die allein oder in Mischungen eingesetzt werrden können. Beispiele für leichter flüchtige Riechstoffe sind Alkylisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Zitral, Zitronellal.

Besonders bevorzugte erfindungsgemäße Deodorant- oder Antitranspirant-Stifte sind dadurch gekennzeichnet, dass mindestens eine Duftstoffkomponente in einer Gesamtmenge von 0,00001 bis 4 Gew.%, bevorzugt 0,5 - 2 Gew.-%, jeweils bezogen auf die Gesamtzusammensetzung, enthalten ist.

### Penetrationskraftwerte

Die erfindungsgemäßen Stiftzusammensetzungen sind weiterhin gekennzeichnet durch einen Penetrationskraftwert im Bereich von 150 - 800 Gramm-Kraft (g-force), bevorzugt 200 - 750 Gramm-Kraft (g-force), besonders bevorzugt 350 - 600 Gramm-Kraft (g-force), bei einer Penetrationstiefe von 5,000 mm. Der Penetrationskraftwert stellt ein Maß für die Härte eines Stiftes (oder aber auch einer festen Cremezusammensetzung) dar und gibt an, mit welcher Maximalkraft eine definierte Messsonde, hier ein Edelstahlkonus mit 45° (Modell TA 15), bis zu einer für die Messung geeigneten Penetrationstiefe, hier bis zu einer Penetrationstiefe von 5,000 mm (fünf komma null null null mm), mit einer Vorschubgeschwindigkeit von 2 mm/Sekunde senkrecht (axial) in die zu vermessende Antitranspirantmasse eingefahren wird. Die Bestimmung des Penetrationskraftwertes wird durchgeführt mit dem TA-XT21 Texture Analyzer der Firma Stable Micro Systems (Vienna Court, Lammas Road, Godalming, Surrey GU7 1YL, England). Die Maximalkraft wird in Gramm-Kraft (g-force) angegeben. Dabei bedeuten niedrigere Werte eine weichere Zusammensetzung, härtere Zusammensetzungen weisen einen höheren Penetrationskraftwert auf. Cremeartige Zusammensetzungen werden häufig mit einer Penetrationstiefe von 10,000 mm (zehn komma null null null mm) vermessen, um genauere Werte zu erhalten. Diese Eindringtiefe kann bei härteren Stiftmassen meist nicht vermessen werden, da hierbei oft schon die Stiftmasse zu brechen beginnt. Eine Verdoppelung der Penetrationstiefe bedeutet etwa eine Verdreifachung bis Vervierfachung des Messwertes der Maximalkraft. Die Messungen erfolgen bei Umgebungsbedingungen von 30°C und 50% relativer Luftfeuchte, die Probentemperatur beträgt 23°C. Die in DE 199 62 878 A1 und DE 199 82 881 A1 offenbarten Antitranspirant-Cremes weisen unter den hier genannten Messbedingungen Penetrationskraftwerte von 9 - 15 Gramm-Kraft (g-force) auf.

### Elektrischer Widerstand

Die erfindungsgemäßen Stiftzusammensetzungen sind weiterhin gekennzeichnet durch einen elektrischen Widerstand von maximal 300 kΩ. Bevorzugt Ist ein elektrischer Widerstand von maximal 100 kΩ, besonders bevorzugt von maximal 80 kΩ. Der Widerstand wird mit einem Multimeter Voltcraft Modell VC820 mit automatischer Messbereichsumschaltung (0-400 Ω / 40MΩ (± 1% + 2dgt)) und zwei Mikrotipp Messfühlern 1,0 mm aus Edelstahl gemessen. Der Elektrodenabstand wird mit einer Millimeterlehre fixiert. Die Messung wird bei Raumtemperatur (22°C) durchgeführt. Dazu werden die Mikrotipp-Elektroden im Abstand von 27,0 mm parallel auf der Millimeterlehre fixiert und mit dem Widerstandsmessgerät verbunden. Die Messung des elektrischen Widerstandes erfolgt direkt an den wasserhaltige Antitranspirantatiften. Dazu wird die üblicherweise gewölbte Oberfläche der Antitranspirantstifte mit einem Messer soweit abgetragen, dass sich eine ebene Schnittfläche ergibt. Unmittelbar danach werden die Messelektroden ca. 5 mm senkrecht in die Stiftmasse eingesteckt. Der Messwert des elektrischen Widerstandes wird nach 30 Sekunden abgelesen. Die Reinigung der Messelektroden erfolgt mit einem alkoholgetränkten Zellstofftuch. Unter den genannten Messbedingungen weist Leitungswasser einen elektrischen Widerstand von 250 kΩ, eine 20 Gew.%ige wässrige Aluminiumchlorohydrat-Lösung 3 kΩ und vollentsalztes Wasser 1,7 MΩ auf.

### Weitere Wirkstoffe

Besonders bevorzugte erfindungsgemäße Deodorant- oder Antitranspirant-Stifte sind dadurch gekennzeichnet, dass weiterhin Pigmente, z. B. Titandioxid, enthalten Ist. Der Pigmentgehalt unterstützt die kosmetische Akzeptanz des Präparates beim Anwender. Weiterhin sind besonders bevorzugte erfindungsgemäße Deodorant- oder Antitranspirant-Stifte dadurch gekennzeichnet, dass sie die üblichen Bestandteile kosmetischer Präparate enthalten, z. B. Farbstoffe, Nanosphären, Konservierungs- und Lichtschutzmittel, Antioxidantien, Enzyme sowie Pflegestoffe. Diese sind in besonders bevorzugten erfindungsgemäßen Deodorant- oder Antitranspirant-Stiften vorzugsweise in einer Menge von 0,001 - 20 Gew.-% enthalten.

### Produktstabilisierung

Besonders bevorzugte erfindungsgemäße Deodorant- oder Antitranspirant-Stifte sind dadurch gekennzeichnet, dass sie zur Produktstabilisierung mindestens eine Radikalfängersubstanz enthalten, besonders bevorzugt eine Substanz mit der INCI-Bezeichnung Tris(tetramethylhydroxypiperidinol)citrate, die z. B. unter dem Handelsnamen Tinogard Q von der Firma Ciba erhältlich ist. Tris(tetramethylhydroxypiperidinol)citrat ist bevorzugt in Mengen von 0,01 - 0,1, besonders bevorzugt 0,025 - 0,05 Gew.-%, bezogen auf das Gesamtgewicht der erfindungsgemäßen Zusammensetzung, enthalten.

Weitere besonders bevorzugte erfindungsgemäße Deodorant- oder Antitranspirant-Stifte sind dadurch gekennzeichnet, dass sie mindestens einen UV-Filter enthalten. Dabei sind die UV-Filter bevorzugt ausgewählt aus Benzotriazolderivaten, insbesondere 2-(5-Chloro-2H-benzotriazol-2-yl)-6-(1,1-dimethylethyl)-4-methyl-phenol (INCl-Bezeichnung: Bumetrizole, erhältlich z. B. unter dem Handelsnamen Tinogard AS von der Firma Ciba), 2,2'-Methylen-bis-(6-(2H-benzotriazol-2-yl)-4-(1,1,3,3-tetramethylbutyl)-phenol) [Tinosorb M (Ciba)] 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS-Nr,: 025973-551), 2-(2'- Hydroxy-5'-octylphenyl)-benzotriazol (CAS-Nr. 003147-75-9), 2-(2'-Hydroxy-5'-methylphenyl)benzotriazol (CAS-Nr. 2440-22-4), 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane, 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin oder auch Aniso Triazin, erhältlich als Tinosorb^{®} S von CIBA), 2,4-Bis-{[4-(3-sulfonato)-2-hydroxy-propyloxy)-2-hydroxy]-phenyl}-6-(4-methoxyphenyl)-1,3,5-triazin-Natriumsalz, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-[4-(2-methoxyethylcarboxyl)-phenylamino]-1,3,5-triazin, 2,4-Bis-{[4-(3-(2-propyloxy)-2-hydroxy-propyloxy)-2-hydroxy]-phenyl}-6-[4-(ethyl-carboxyl)-phenylamino]-1,3,5-triazin, 2,4-Bis-{[4-(2-ethylhexyloxy)-2-hydroxy]-phenyl}-6-(1-methyl-pyrrol-2-yl)-1,3,5-triazin, 2,4-Bis- {[4-tris(trimethylsiloxy-silylpropyloxy)-2-hydroxy]-phenyl}-6-(4-methoxyphenyl)-1,3,5-triazin, 2,4-Bis-{[4-(2-methylpropenyloxy)-2-hydroxy]-phenyl}-6-(4-methoxyphenyl)-1,3,5-triazin, 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin sowie Mischungen der genannten Komponenten. Weiterhin ist der Zusatz wasserlöslicher UV-Filter bevorzugt. Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, insbesondere die Sulfonsäure selbst mit der INCI Bezeichnung Phenylbenzimidazole Sulfonic Acid (CAS.-Nr. 27503-81-7), die beispielsweise unter dem Handelsnamen Eusolex 232 bei Merck oder unter Neo Heliopan Hydro bei Symrise erhältlich ist, und das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonate (CAS-Nr.: 180898-37-7), das beispielsweise unter dem Handelsnamen Neo Heliopan AP bei Symrise erhältlich ist, Sulfonsäurederivate von Benzophenonen, vorzugsweise 2-Hydroxy-4-methoxybenzophenon-5-sulfonsäure und ihre Salze und Sulfonsäurederivate des 3-Benzylidencamphers, wie z. B. 4-(2-Oxo-3-bornylidenmethyl)-benzolsulfonsäure.

Weitere besonders bevorzugte erfindungsgemäße Deodorant- oder Antitranspirant-Stifte sind dadurch gekennzeichnet, dass sie zur Produktstabilisierung den Radikalfänger Tris(tetramethylhydroxypiperidinol)citrat und den UV-Filter Bumetrizole enthalten. Bumetrizole ist bevorzugt in Mengen von 0,01 - 0,1, besonders bevorzugt 0,025 - 0,05 Gew.-%, bezogen auf das Gesamtgewicht der erfindungsgemäßen Zusammensetzung, enthalten.

Weitere besonders bevorzugte erfindungsgemäße Deodorant- oder Antitranspirant-Stifte sind dadurch gekennzeichnet, dass sie zur Produktstabilisierung mindestens eine komplexbildende Substanz enthalten. Als komplexbildende Substanz besonders bevorzugt ist Ethylendiamintetraessigsäure (EDTA) und ihre Natriumsalze, wie sie beispielsweise unter dem Handelsnamen Trilon B von der Firma BASF erhältlich ist, weiterhin Nitrilotriessigsäure (NTA) und ihre Natriumsalze, β-Alanindiessigsäure und ihre Salze und Phosphonsäuren und deren Salze. Die mindestens eine komplexbildende Substanz ist bevorzugt in einer Gesamtmenge von 0,01 - 0,5 Gew.-%, besonders bevorzugt 0,08 - 0,2 Gew.-%, bezogen auf das Gesamtgewicht der erfindungsgemäßen Zusammensetzung, enthalten.

Weitere außerordentlich bevorzugte erfindungsgemäße Deodorant- oder Antitranspirant-Stifte sind dadurch gekennzeichnet, dass sie mindestens eine Radikalfängersubstanz und mindestens eine Substanz, ausgewählt aus UV-Filtern und komplexbildenden Substanzen, enthalten.

Weitere außerordentlich bevorzugte erfindungsgemäße Deodorant- oder Antitranspirant-Stifte sind dadurch gekennzeichnet, dass sie mindestens eine Radikalfängersubstanz, mindestens einen UV-Filter und mindestens eine komplexbildende Substanz enthalten.

Weitere außerordentlich bevorzugte erfindungsgemäße Deodorant- oder Antitranspirant-Stifte sind dadurch gekennzeichnet, dass sie Tris(tetramethylhydroxypiperidinol)citrate, Bumetrizole und Ethylendiamintetraessigsäure, letztere gegebenenfalls als Natriumsalz, enthalten.

### Haarwuchsinhibitoren

Weitere besonders bevorzugte erfindungsgemäße Deodorant- oder Antitranspirant-Stifte sind dadurch gekennzeichnet, dass sie mindestens eine den Haarwuchs inhibierende Substanz enthalten. Geeignete Substanzen, die den Haarwuchs inhibieren, sind insbesondere ausgewählt aus Eflornithin, Wirkstoffkombinationen aus Sojaproteinhydrolysat, Harnstoff, Menthol, Salicylsäure und Extrakten aus Hypericum Perforatum, Hamamelis Virginiana, Arnica Montana und der Rinde von Salix Alba, wie sie beispielsweise und bevorzugt in dem Rohstoff Pilinhib^{®} Veg LS 9109 von Laboratoires Sérobiologiques mit der INCI-Deklaration "Propylene glycol, Hydrolyzed Soy Protein, Hypericum Perforatum Extract, Hamamelis Virginiana Extract, Arnica Montana Flower Extract, Urea, Salix Alba Bark Extract, Menthol, Salicylic acid" enthalten ist, weiterhin Wirkstoffkombinationen aus Extrakten aus Epilobium Angustifolium, den Samen von Cucurbita pepo (Kürbis) und den Früchten von Serenoa serrulata, wie sie beispielsweise und bevorzugt in dem Rohstoff ARP 100 von Greentech S.A./Rahn mit der INCI-Deklaration Water, Alcohol, Serenoa Serrulata Fruit Extract, Epilobium Angustifolium Extract, Cucurbita Pepo (Pumpkin) Seed Extract" enthalten sind, weiterhin Wirkstoffkombinationen aus Xylitol und Extrakten von Citrus Medica Limonum (Lemon) Fruit, Carica Papaya (Papaya) und Olivenblättern, wie sie beispielsweise und bevorzugt in dem Rohstoff Xyleine von Impag/ Seporga mit der INCI-Deklaration ,,Xylitol and Citrus Medica Limonum (Lemon) Fruit Extract and Carica Papaya (Papaya) Fruit Extract and Olea europaea (olive) leaf extract" enthalten sind, weiterhin Wirkstoffkombinationen aus Humulus Lupulus, Viscum Album, Salvia Officinalis, Carica Papaya und Thuya Occidentalis, wie sie beispielsweise und bevorzugt in dem Rohstoff Plantafluid Komplex AH der Firma Plantapharm mit der INCI-Deklaration ,,Aqua, Propylene Glycol, Humulus Lupulus, Viscum Album, Salvia Officinalis, Carica Papaya, Thuya Occidentalis" enthalten sind, sowie Extrakten aus Larrea divaricata, wie sie beispielsweise und bevorzugt in dem Rohstoff Capislow von Sederma, der Lecithinvesikel mit einem hydroglycolischen Extrakt aus Larrea divaricata enthält, enthalten sind.

Die erfindungsgemäßen Zusammensetzungen enthalten mindestens eine den Haarwuchs inhibierende Substanz vorzugsweise in einer Menge von 0,1 -10 Gew.-%, bevorzugt 0,5 - 5 Gew.-% und besonders bevorzugt 1 - 4 Gew.-%, jeweils bezogen auf das Gewicht des Rohstoffs tel quel und das Gesamtgewicht der erfindungsgemäßen Zusammensetzung.

### Konservierungsstoffe

Den erfindungsgemäßen Zusammensetzungen können auch die üblichen Konservierungsstoffe zugesetzt werden, um den Verderb des Produktes durch mikrobielles Wachstum zu verhindern. Zahlreiche Konservierungsstoffe haben zwangsläufig auch desodorierende Eigenschaften, so dass einige Stoffe beiden Gruppen angehören. Für Kosmetika als Konservierungsstoffe bevorzugt geeignet sind beispielsweise Benzoesäure und deren Derivate (z. B. Propyl-, Phenyl- und Butylbenzoat, Ammonium-, Natrium, Kalium- und Magnesiumbenzoat), Propionsäure und deren Derivate (z. B. Ammonium-, Natrium-, Kalium-, und Magnesiumpropionat), Salicylsäure und deren Derivate (z. B. Natrium-, Kalium- und Magnesiumsalicylat), 4-Hydroxybenzoesäure und deren Ester und Alkalimetallsalze (z. B. Methyl-, Ethyl-, Propyl-, Isopropyl-, Butyl-, Isobutyl-, Isodecyl-, Phenyl-, Phenoxyethyl- und Benzylparaben, Hexamidinparaben und -diparaben, Natrium- und Kaliumparaben, Natrium- und Kaliummethylparaben, Kaliumbutylparaben, Natrium- und Kaliumpropylparaben), Alkohole und deren Salze (z.B. Ethanol, Propanol, Isopropanol, Benzylalkohol, Phenethylalkohol, Phenol, Kaliumphenolat, Phenoxyethanol, Phenoxyisopropanol, o-Phenylphenol), Guajacol und dessen Derivate, Chlorhexidin und dessen Derivate (z. B. Chlorhexidindiacetat, -digluconat, und -dihydrochlorid), Hydantoin und dessen Derivate (z. B. DEDM- und DMDM-Hydantoin, DEDM-Hydantoindilaurat), Harnstoff und Harnstoffderivate (z. B. Diazolidinylharnstoff, Imidazolidinylharnstoff), Ferulasäure und deren Derivate (z. B. Ethylferulat), Sorbinsäure und deren Derivate (z. B. Isopropylsorbat, TEA-Sorbat, Natrium-, Kalium-, und Magnesiumsorbat), Isothiazol- und Oxazolderivate (z. B. Methylisothiazolinon, Methylchloroisothiazolinon, Dimethyloxazolidin), quartäre Ammoniumverbindungen (z. B. Polyquaternium-42, Quaternium-8, Quaternium-14, Quaternium-15), Carbamate (z. B. Iodopropynylbutylcarbamat), Formaldehyd und Natriumformat, Glutaraldehyd, Glyoxal, Hexamidin, Dehydracetsäure, 2-Bromo-2-nitropropan-1,3-diol, Isopropylkresol, Methyldibromoglutaronitril, Polyaminopropylbiguanid, Natriumhydroxymethylglycinat, Natriumphenolsulfonat, Triclocarban, Triclosan, Zinkpyrithion sowie diverse Peptid-Antibiotika (z. B. Nisin). Erfindungsgemäß bevorzugte Konservierungsmittel sind Phenoxyethanol, die Ester der 4-Hydroxybenzoesäure, insbesondere Methyl-, Ethyl-, Propyl-, Isopropyl-, Butyl- und Isobutylparaben sowie Iodopropynylbutylcarbamat.

Die Menge der Konservierungsmittel in den bevorzugten erfindungsgemäßen Zusammensetzungen beträgt 0,001 - 10 Gew.-%, vorzugsweise 0,01 - 5 Gew.-% und insbesondere 0,1 - 3 Gew.-%, bezogen auf das Gesamtgewicht der Zusammensetzung.

Prinzipiell ist der Gegenstand der vorliegenden Erfindung auch auf andere kosmetische Stiftzusammensetzungen auszuweiten, die keine Deodorant- oder Antitranspirant-Stifte darstellen. Ein Gehalt an Deodorant- oder Antitranspirant-Wirkstoffen ist in derartigen Stiften nicht obligatorisch. Entsprechende Stifte können beispielsweise als Lippenstift oder als Abdeckstift konfektioniert und durch topische Applikation auf die Haut verwendet werden.

Ein weiterer Gegenstand der vorliegenden Erfindung ist ein kosmetisches, nicht-therapeutisches Verfahren zur Verringerung von Körpergeruch, das dadurch gekennzeichnet ist, dass eine kosmetische Deodorant- und/oder Antitranspirant-Zusammensetzung gemäß einem der Patentansprüche 1 - 15 auf die Haut, insbesondere auf die Haut der Achselhöhlen, aufgetragen wird.

Ein weiterer Gegenstand der vorliegenden Erfindung ist ein Verfahren zur Herstellung eines Deodorant- oder Antitranspirant-Stiftes gemäß einem der Ansprüche 1 - 15, wobei die Wachs- und Ölkomponenten zusammen mit dem/den Öl-in-Wasser- und dem/den Wasser-in-Öl-Emulgator/en auf 90 - 95°C erhitzt und aufgeschmolzen werden, danach das ebenfalls auf 90 - 95°C erhitzte Wasser mit den wasserlöslichen Wirk- und Inhaltsstoffen unter kräftigem Rühren zugegeben wird, gegebenenfalls weitere Inhaltsstoffe beigemischt werden, die Mischung bis auf eine geeignete Abfülltemperatur abgekühlt, in geeignete Spenderformen abgefüllt und durch statisches Abkühlen (ohne weiteres Rühren) auf Raumtemperatur verfestigt wird.

Die nachfolgenden Beispiele sollen den Gegenstand der Erfindung verdeutlichen, ohne ihn hierauf zu beschränken.

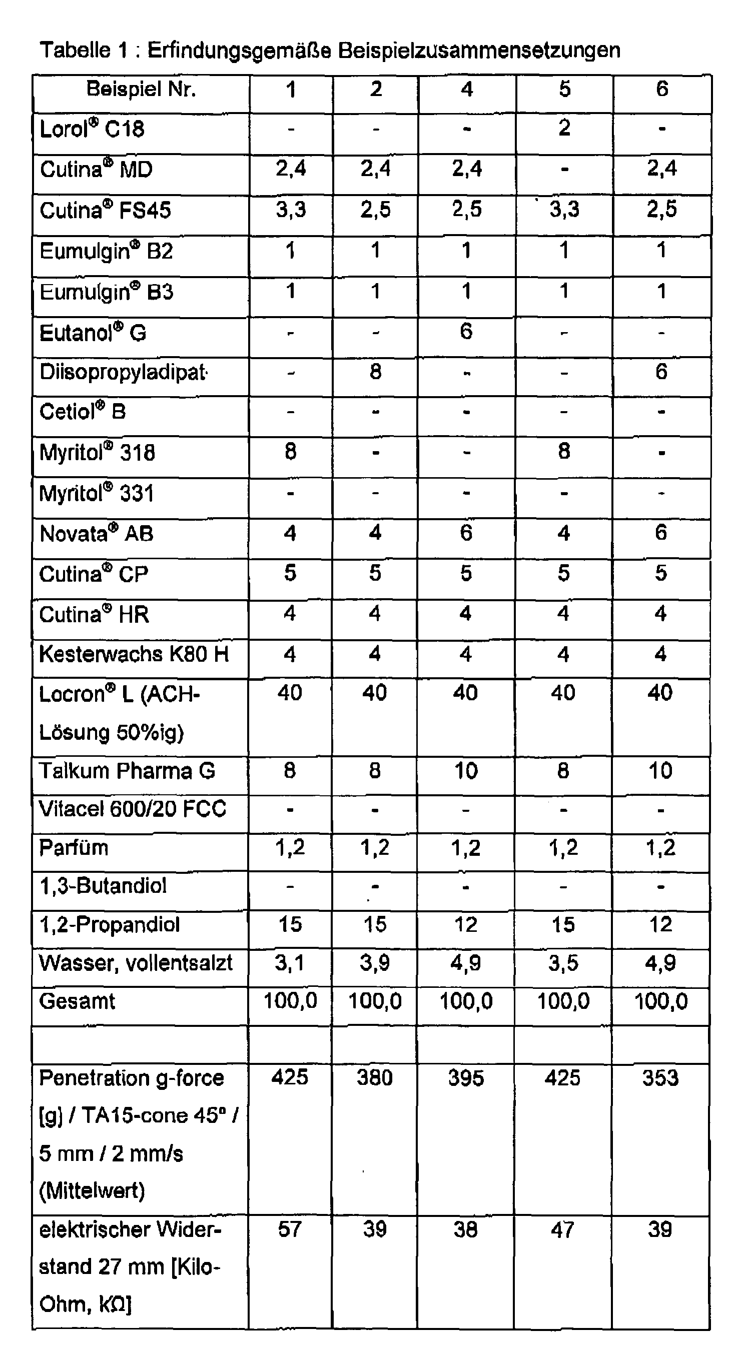

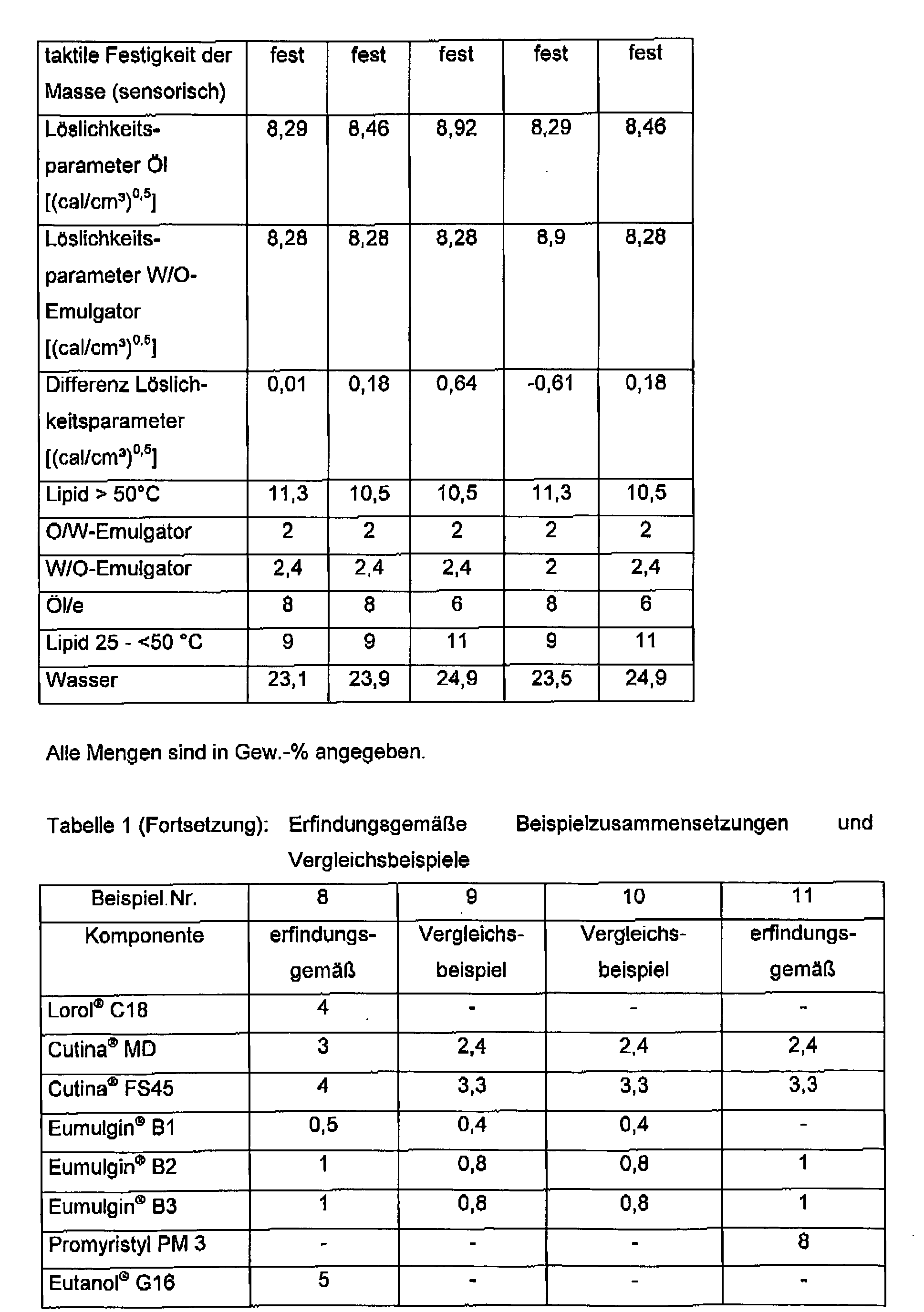

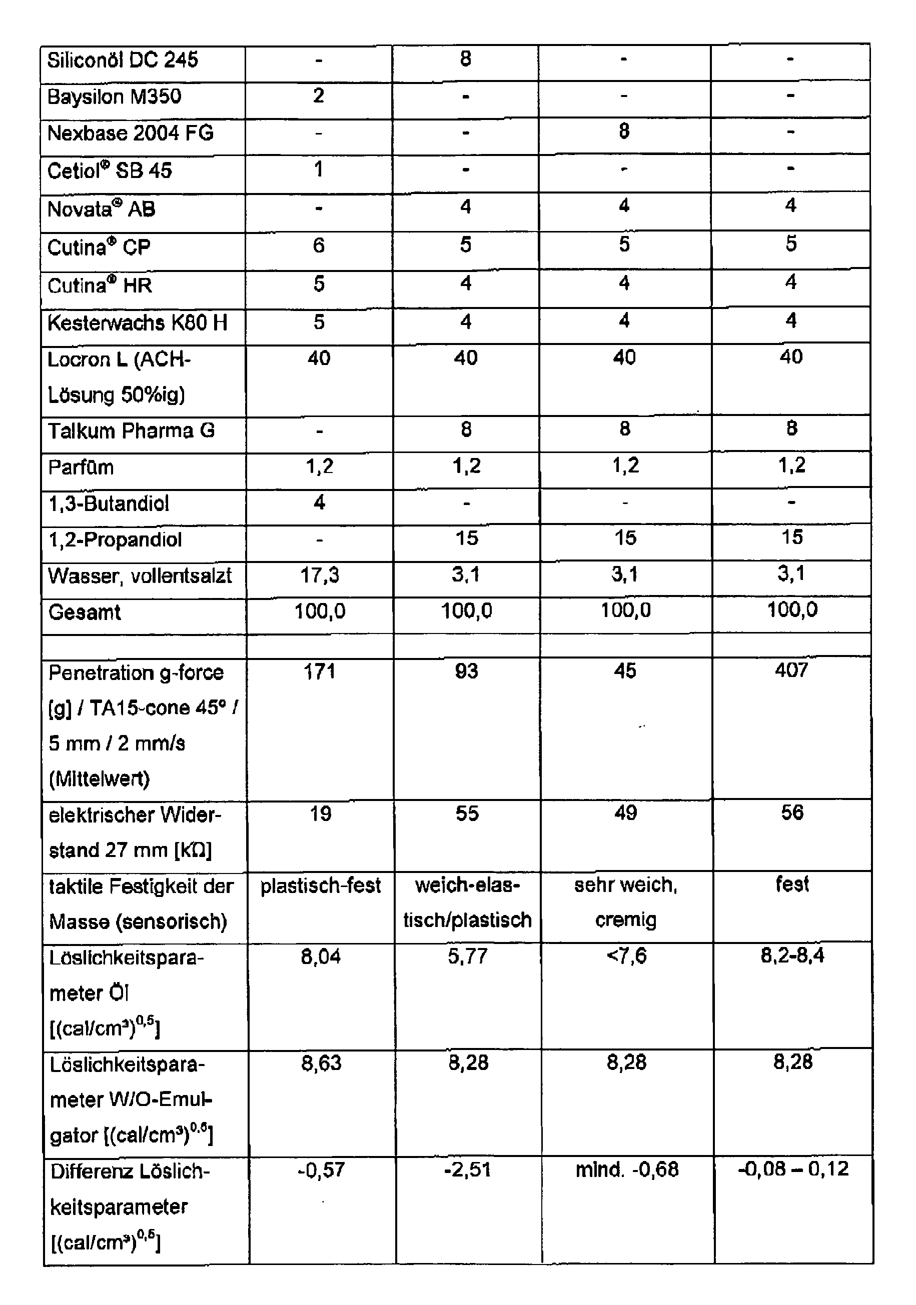

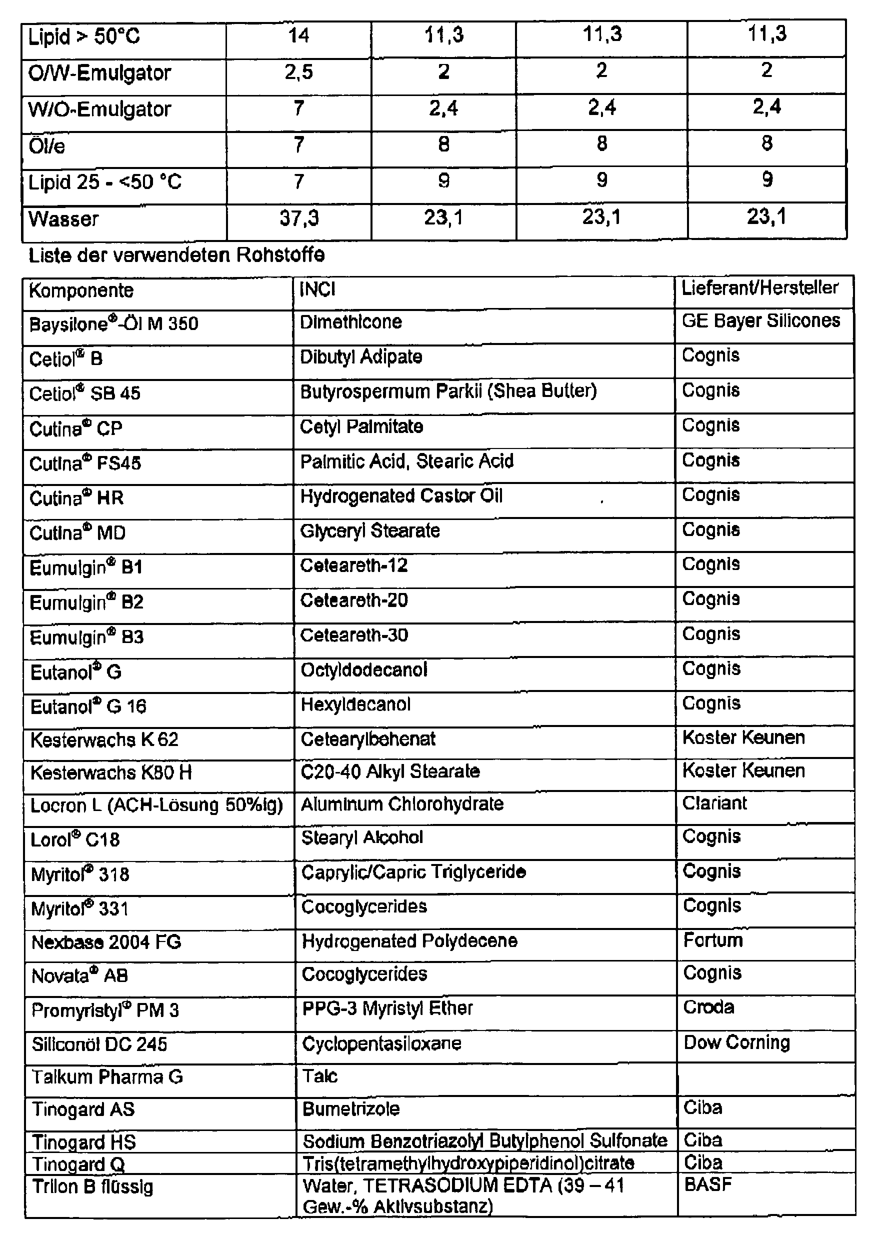

## Patentansprüche

1. Deodorant- oder Antitranspirant-Stift in Form einer Öl-in-Wasser-Dispersion, enthaltend
a) insgesamt 4-20 Gew.-% mindestens einer Lipid- oder Wachskomponente mit einem Schmeizpunkt > 50°C, die nicht den Komponenten b) oder c) zuzurechnen ist,
b) ein nichtionisches Öl-in-Wasser- Emulgatorgemisch mit einem mittleren HLB-Wert im Bereich von 10-19, enthaltend, bezogen auf die gesamtzusammensetzung 0,5 -10 Gew.-% mindestens eines nichtionischen Öl-in-Wasser-Emulgators mit einem HLB-Wert von mehr als 7,
c) insgesamt 0,1 -15 Gew.-% mindestens eines nichtionischen Wasser-in-Öl-Emulgators mit einem HLB-Wert größer 1,0 und kleiner/gleich 7,0, der mit Wasser allein oder mit Wasser in Gegenwart eines hydrophilen Emulgators flüssigkristalline Strukturen ausbilden kann und der ausgewählt ist aus
- linearen. gesättigten: C₁₂ - C₃₀-Alkanolen.
- Ethylenglycolmono- und diestern von linearen, gesättigten und ungesättigten C₁₂ - C₃₀-Carbonsäuren; die hydroxyliert sein können
- Glycerinmono- und diestern von linearen, gesättigten und ungesättigen C₁₂ - C₃₀-Carbonsäuren, die hydroxyliert sein können:
- Propylenglycolmono- und -diestern von linearen, gesättigten und ungesättig-ten C₁₂-C₃₀-Carbonsäuren, die hydroxyliert sein können.
- Sorbitanmono-, -di- und -triestern von linearen, gesättigten und ungesättigten C₁₂ - C₃₀-Carbon-säuren, die hydroxyliert sein können
- Pentaerythritylmono-, -di-, -tri- und -tetraestern von linearen, gesättigten und ungesättigten C₁₂ - C₃₀-Carbonsäuren, die hydroxyliert sein können,
- Methylglucosemono- und -diestern von linearen gesättigten und ungesättigten C₁₂ - C₃₀-Carbonsäuren, die hydroxyliert sein können
- Sterinen,
- Alkanolen und Carbonsäuren mit jeweils 8 - 24 C-Atomen, insbesondere mit 16 - 22 C-Atomen, in der Alkylgruppe und 1 - 4 Ethylenoxid-Einheiten pro Molekül, die einen HLB-Wert größer 1,0 und kleiner/gleich 7,0 aufweisen,
- Glycerinmonoethern gesättigter und/oder ungesättigter, verzweigten und/oder unverzweigter Alkohole einer Kettenlänge von 8 - 30, insbesondere 12 -18 Kohlenstoffatomen.
- Partialestern von Polyglycerinen mit n = 2 bis 10 Glycerineinheiten und mit 1 bis 5 gesättigten oder ungesättigten, linearen oder verzweigten, gegebenenfalls hydroxylierten C₈ - C₃₀-Fettsäureresten verestert, sofern sie einen HLB-Wert von kleiner/gleich 7 aufweisen,
- sowie Mischungen der vorgenannten Substanzen:
als Konsistenzgeber und/oder Wasserbinder,
d) insgesamt 3-20 Gew.-% mindestens eines bei 20 ° C flüssigen Öls, das keine Duftstoffkomponente und kein etherisches Öl darstellt, wobei der (mittlere) Löslichkeitsparameter der Gesamtheit der enthaltenen Öle d) in Gegenwart von linearen gesättigten Fettalkoholen mit einer Kettenlänge von mindestens 8 Kohlenstoffatomen um maximal -2,93 (J/cm³)^{0,5} (-0,7 (cal/cm³)^{0,5}) bzw. maximal +2,93 (J/cm³)^{0,5} (+0,7 (cal/cm³)^{0,5}), und in Gegenwart von Wasser-in-Öl-Emulgatoren, die von linearen gesättigten Fettalkoholen mit einer Kettenlänge von mindestens 8 Kohlenstoffatomen verschieden sind, in Abwesenheit von linearen gesättigten Fettalkoholen mit einer Kettenlänge von mindestens 8 Kohlenstoffatomen um maximal -1,67 (J/cm³)^{0,5} (-0,4 (cal/cm³)^{0,5}) bzw. maximal +2,93 (J/cm³)^{0,5} (+0,7 (cal/cm³)^{0,5}) vom (mittleren) Löslichkeitsparameter des Wasser-in-Öl-Emulgators/der Wasser-in-Öl-Emulgatoren abweicht,
e) insgesamt 3-25 Gew.-% mindestens eines wasserlöslichen mehrwertigen C₂ - C₉-Alkanols mit 2 - 6 Hydroxylgruppen und/oder mindestens eines wasserlöslichen Polyethylenglycols mit 3 - 20 Ethylenoxid-Einheiten,
f) 10 bis weniger als 50 Gew.-% Wasser, bezogen auf die Gesamtzusammensetzung,
g) mindestens einen Deodorant- oder Antitranspirant-Wirkstoff, wobei der Stift
h) einen Penetrationskraftwert im Bereich von 150 - 800 Gramm-Kraft (g-force) bei einer Penetrationstiefe von 5,000 mm und
i) einen elektrischen Widerstand von maximal 300 kΩ (Kiloohm), gemessen nach dem in der Beschreibung offenbarten Verfahren,
aufweist, und
j) die Verfestigung des Deodorant- oder Antitranspirant-Stifts nicht auf der Basis von Seifengelen beziehungsweise Fettsäuresalz-Gelen und nicht auf der Basis von anorganischen und/oder organischen polymeren Hydrogelbildnern erfolgt, wobei unter Fettsäuren Alkan-, Alken- und Alkinsäuren mit mindestens 4 Kohlenstoffatomen verstanden werden,
wobei der Löslichkeitsparameter nach einer Methode bestimmt wird, nach der Diisopropyladipat einen Löslichkeitsparameter von 35,42 (J/cm³)^{0,5} (8,46 (cal/cm³)^{0,5}) und Octyldodecanol einen Löslichkeitsparameter von 37,35 (J/cm³)^{0,5} (8,92 (cal/cm³)^{0,5}) aufweisen,
wobei alle hMengenangaben auf die Gesamtzusammensetzung bezogen sind.

2. Deodorant- oder Antitranspirant-Stift gemäß. Anspruch 1, **dadurch gekennzeichnet, dass** die Lipid- oder Wachskomponente a) ausgewählt ist aus Estern aus einem gesättigten, einwertigen C₁₆-C₆₀-Alkanol und einer gesättigten C₈-C₃₆-Monocarbonsäure, insbesondere Cetylbehenat, Stearylbehenat und C₂₀-C₄₀-Alkylstearat, Glycerintriestern von gesättigten linaaren C₁₂-C₃₀-Carbonsäuren, die hydroxyliert sein können, Candelillawachs, Carnaubawachs, Bienenwachs, gesättigten linearen C₁₄ - C₃₀-Carbonsäuren sowie Mischungen der vorgenannten Substanzen.

3. Deodorant- oder Antitranspirant-Stift gemäß einem der vorstehenden Ansprüche, **dadurch gekennzeichnet, dass** die Lipid- oder Wachskomponente a) insgesamt in Mengen von 8 - 15 Gew.-%, bezogen auf die Gesamtzusammensetzung, enthalten ist.

4. Deodorant- oder Antitranspirant-Stift gemäß Anspruch 2, **dadurch gekennzeichnet, dass** der/die Ester aus einem gesättigten, einwertigen C₁₆-C₆₀-Alkanol und einer gesättigten C₈-C₃₆-Monocarbonsäure in Mengen von 2 - 10 Gew.-%, bevorzugt 2 - 6 Gew.-%, bezogen auf die Gesamtzusammensetzung, enthalten ist/sind.

5. Deodorant- oder Antitranspirant-Stift gemäß einem der vorstehenden Ansprüche,
**dadurch gekennzeichnet, dass** der nichtionische Öl-in-Wasser-Emulgator b) mit einem HLB-Wert von mehr als 7 ausgewählt ist aus
- ethoxylierten C₈-C₂₄-Alkanolen mit durchschnittlich 10 -100 Mol Ethylenoxid pro Mol,
- ethoxylierten C₈-C₂₄-Carbonsäuren mit durchschnittlich 10- 100 Mol Ethylenoxid pro Mol,
- Silicon-Copolyolen mit Ethylenoxid-Einheiten oder mit Ethylenoxid- und Propylenoxid-Einheiten,
- Alkylmono- und -oligoglycosiden mit 8 bis 22 Kohlenstoffatomen im Alkylrest und deren ethoxylierten Analoga,
- ethoxylierten Sterinen,
- Partialestern von Polyglycerinen mit n = 2 bis 10 Glycerineinheiten und mit 1 bis 4 gesättigten oder ungesättigten, linearen oder verzweigten, gegebenenfalls hydroxylierten C₈- C₃₀-Fettsäureresten verestert, sofern sie einen HLB-Wert von mehr als 7 aufweisen,
- sowie Mischungen der vorgenannten Substanzen.

6. Deodorant- oder Antitranspirant-Stift gemäß einem der vorstehenden Ansprüche, **dadurch gekennzeichnet, dass** der Öl-in-Wasser-Emulgator b) in einer Gesamtmenge von 1 - 4 Gew.-%, bezogen auf die Gesamtzusammensetzung, enthalten ist.

7. Deodorant- oder Antitranspirant-Stift gemäß einem der vorstehenden Ansprüche, **dadurch gekennzeichnet, dass** der Wasser-in-Öl-Emulgator c) in einer Gesamtmenge von 0,5- 8 Gew.-%, und bevorzugt 1 - 4 Gew.%, bezogen auf das Gesamtgewicht der Zusammensetzung, enthalten ist.

8. Deodorant- oder Antitranspirant-Stift gemäß einem der vorstehenden Ansprüche, **dadurch gekennzeichnet, dass** das bei 20 ° C flüssige Öl d) ausgewählt ist aus
- verzweigten gesättigten oder ungesättigten Fettalkoholen mit 6 - 30 Kohlenstoff atomen,
- Triglyceriden von linearen oder verzweigten, gesättigten oder ungesättigten, gegebenenfalls hydroxylierten C₈₋₃₀-Fettsäuren,
- Dicarbonsäureestern von linearen oder verzweigten C₂-C₁₀-Alkanolen,
- Estern von verzweigten gesättigten oder ungesättigten Fettalkoholen mit 2 - 30 Kohlenstoffatomen mit linearen oder verzweigten gesättigten oder ungesättigten Fettsäuren mit 2 - 30 Kohlenstoffatomen, die hydroxyliert sein können,
- Anlagerungsprodukten von 1 bis 5 Propylenoxid-Einheiten an ein- oder mehr wertige C₈₋₂₂-Alkanole,
- Anlagerungsprodukten von mindestens 6 Ethylenoxid- und/oder Propylenoxid-Einheiten an ein- oder mehrwertige C₃₋₂₂-Alkanole,
- C₈-C₂₂-Fettaikohoiestern einwertiger oder mehrwertiger C₂-C₇-Hydroxycarbonsäuren,
- symmetrischen, unsymmetrischen oder cyclischen Estern der Kohlensäure mit Fettalkoholen,
- den Estern von Dimeren ungesättigter C₁₂C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen,
- sowie Mischungen der vorgenannten Substanzen.

9. Deodorant- oder Antitranspirant-Stift gemäß einem der vorstehenden Ansprüche, **dadurch gekennzeichnet, dass** das bei 20 ° C flüssige Öl d) in einer Gesamtmenge von 14 Gew.-%, bezogen auf das Gesamtgewicht der Zusammensetzung, enthalten ist.

10. Deodorant- oder Antitranspirant-Stift gemäß einem der vorstehenden Ansprüche, **dadurch gekennzeichnet, dass** die bei 20 ° C flüssigen Öle d) maximal 20 Gew.-% Öl/e, bezogen auf das Gesamtgewicht an bei 20 ° C flüssigen Ölen, enthalten, deren Löslichkeitsparameter um mehr als -1,67 (J/cm³)^{0,5} bzw. -2,93 (J/cm³)^{0,5} (-0,4 bzw. -0,7 (cal/cm³)^{0,5}) oder um mehr als +2,93 (J/cm^{3)0,5} (+0,7 (cal/cm³)^{0,5}) vom (mittleren) Löslichkeitsparameter des Wasser-in-Öl-Emulgators/der Wasser-in-Öl-Emulgatoren c) abweicht.

11. Deodorant- oder Antitranspirant-Stift gemäß einem der vorstehenden Ansprüche, **dadurch gekennzeichnet, dass** keine bei 20 ° C flüssigen Öle enthalten sind, deren Löslichkeitsparameter um mehr als ± 4,1868 (J/cm³)^{0,5} (± 1,0 (cal/cm³)^{0,5}) vom (mittleren) Löslichkeitsparameter des Wasser-in-Öl-Emulgators/der Wasser-in-Öl-Emulgatoren c) abweicht.

12. Deodorant- oder Antitranspirant-Stift gemäß einem der vorstehenden Ansprüche, **dadurch gekennzeichnet, dass** das wasserlösliche mehrwertige C₂-C₉-Alkanol mit 2 - 6 Hydroxylgruppen und/oder das wasserlösliche Polyethylenglycol mit 3 - 20 Ethylenoxid-Einheiten insgesamt in Mengen von 8 - 18 Gew.-%, bezogen auf die Gesamtzusammensetzung, enthalten ist.

13. Deodorant- oder Antitranspirant-Stift gemäß einem der vorstehenden Ansprüche, **dadurch gekennzeichnet, dass** weiterhin mindestens eine Lipid- oder Wachskomponente mit einem Schmelzpunkt im Bereich von 25 - < 50°C, ausgewählt aus Kokosfettsäureglycerinmono-, -di- und -triestern, Butyrospermum Parkii (Shea Butter) und Estern von gesättigten, einwertigen C₈-C₁₈-Alkoholen mit gesättigten C₁₂-C₁₂-Monocarbonsäuren sowie Mischungen dieser Substanzen, enthalten ist.

14. Deodorant- oder Antitranspirant-Stift gemäß Anspruch 13, **dadurch gekennzeichnet, dass** die Lipid- oder Wachskomponente mit einem Schmelzpunkt im Bereich von 25 - < 50°C in Mengen von 0,01 bis 20 Gew.-%, bevorzugt 3 - 20 Gew.%, besonders bevorzugt 5 - 18 Gew.-% und außerordentlich bevorzugt 6 -15 Gew.-%, bezogen auf die Gesamtzusammensetzung, enthalten ist.

15. Deodorant- oder Antitranspirant-Stift gemäß einem der vorstehenden Ansprüche, **dadurch gekennzeichnet, dass** 10 bis weniger als 30 Gew.% Wasser, bezogen auf die Gesamtzusammensetzung, enthalten sind.

## Claims

1. A deodorant or antiperspirant stick in the form of an oil-in-water dispersion, containing
a) in total 4-20 wt.% of at least one lipid or wax component with a melting point of >50°C, which does not fall within the class of components b) or c),
b) a nonionic oil-in-water emulsifier mixture with an average HLB value in the range from 10-19 containing, relative to the total composition, 0.5-10 wt.% of at least one nonionic oil-in-water emulsifier with an HLB value of more than 7,
c) in total 0.1-15 wt.% of at least one nonionic water-in-oil emulsifier with an HLB value of greater than 1.0 and less than or equal to 7.0 which is capable of forming liquid crystalline structures with water alone or with water in the presence of a hydrophilic emulsifier and which is selected from
- linear, saturated C₁₂-C₃₀ alkanols,
- ethylene glycol mono- and diesters of linear, saturated and unsaturated C₁₂-C₃₀ carboxylic acids, which may be hydroxylated,
- glycerol mono- and diesters of linear, saturated and unsaturated C₁₂-C₃₀ carboxylic acids, which may be hydroxylated;
- propylene glycol mono- and diesters of linear, saturated and unsaturated C₁₂-C₃₀ carboxylic acids, which may be hyd roxylated;
- sorbitan mono-, di- and triesters of linear, saturated and unsaturated C₁₂-C₃₀ carboxylic acids, which may be hydroxylated,
- pentaerythrityl mono-, di-, tri- and -tetraesters of linear, saturated and unsaturated C₁₂-C₃₀ carboxylic acids, which may be hydroxylated:
- methyl glucose mono- and diesters of linear, saturated and unsaturated C₁₂-C₃₀ carboxylic acids, which may be hydroxylated,
- sterols,
- alkanols and carboxylic acids with in each case 8-24 C atoms, in particular with 16-22 C atoms, in the alkyl group and 1-4 ethylene oxide units per molecule, which have an HLB value of greater than 1.0 and less than or equal to 7.0,
- glycerol monoethers of saturated and/or unsaturated, branched and/or unbranched alcohols of a chain length of 8-30, in particular 12-18, carbon atoms,
- partial esters of polyglycerols with n = 2 to 10 glycerol units and esterified with 1 to 5 saturated or unsaturated, linear or branched, optionally hydroxylated C₈-C₃₀ fatty acid residues, providing they have an HLB value of less than or equal to 7,
- and mixtures of the above-stated substances;
as a consistency provider and/or water-binding agent,
d) in total 3-20 wt.% of at least one oil which is liquid at 20°C, which is neither a scent component nor an essential oil, wherein the (average) solubility parameter of the entirety of the oils d) contained therein deviates, in the presence of linear saturated fatty alcohols with a chain length of at least 8 carbon atoms, by at most -2.93 (J/cm³)^{0.5} (-0.7 (cal/CM³)^{0.5}) or at most +2.93 (J/cm³)^{0.5} (+0.7 (cal/cm³)^{0.5}) and, in the presence of water-in-oil emulsifiers, which differ from linear saturated fatty alcohols with a chain length of at least 8 carbon atoms, in the absence of linear saturated fatty alcohols with a chain length of at least 8 carbon atoms, by at most -1.67 (J/cm³)^{0.5} (-0.4 (cal/cm³)^{0.5}) or at most +2.93 (J/cm³)^{0.5} (+0.7 (cal/cm³)^{0.5}) from the (average) solubility parameter of the water-in-oil emulsifier(s),
e) in total 3-25 wt.% of at least one water-soluble polyhydric C₂-C₉ alkanol with 2-6 hydroxyl groups and/or at least one water-soluble polyethylene glycol with 3-20 ethylene oxide units,
f) 10 to less than 50 wt.% of water, relative to the total composition,
g) at least one deodorant or antiperspirant active ingredient, wherein the stick exhibits
h) a penetration force value in the range from 150-800 gram force (g force) at a penetration depth of 5.000 mm and
i) an electrical resistance of at most 300 kΩ (kiloohms), measured by the method disclosed in the description
and
j) solidification of the deodorant or antiperspirant stick does not proceed on the basis of soap gels or fatty acid salt gels nor on the basis of inorganic and/or organic polymeric hydrogel formers, wherein fatty acids are taken to mean alkanoic, alkenoic and alkynoic acids with at least 4 carbon atoms,
wherein the solubility parameter is determined by a method according to which diisopropyl adipate exhibits a solubility parameter of 35.42 (J/cm³)^{0.5} (8.46(cal/cm³)^{0.5}) and octyldodecanol a solubility parameter of 37.35 (J/cm³)^{0.5} (8.92 (cal/cm3)^{0.5}),
wherein all the quantities stated relate to the total composition.

2. A deodorant or antiperspirant stick according to claim 1, **characterised in that** the lipid or wax component a) is selected from esters of a saturated, monohydric C₁₆-C₆₀ alkanol and a saturated C₈-C₃₆ monocarboxylic acid, in particular cetyl behenate, stearyl behenate and C₂₀-C₄₀ alkyl stearate, glycerol triesters of saturated linear C₁₂-C₃₀ carboxylic acids, which may be hydroxylated, candelilla wax, carnauba wax, beeswax, saturated linear C₁₄-C₃₆ carboxylic acids and mixtures of the above-stated substances.

3. A deodorant or antiperspirant stick according to one of the preceding claims, **characterised in that** the lipid or wax component a) is present in total in quantities of 8-15 wt.%, relative to the total composition.

4. A deodorant or antiperspirant stick according to claim 2, **characterised in that** the ester(s) prepared from a saturated, monohydric C₁₆-C₆₀ alkanol and a saturated C₈-C₃₆ monocarboxylic acid is/are present in quantities of 2-10 wt.%, preferably of 2-6 wt.%, relative to the total composition.

5. A deodorant or antiperspirant stick according to one of the preceding claims, **characterised in that** the nonionic oil-in-water emulsifier b) with an HLB value of more than 7 is selected from
- ethoxylated C₈-C₂₄ alkanols with on average 10-100 mol of ethylene oxide per mol,
- ethoxylated C₈-C₂₄ carboxylic acids with on average 10-100 mol of ethylene oxide pro mol,
- silicone copolyols with ethylene oxide units or with ethylene oxide and propylene oxide units,
- alkyl mono- and oligoglycosides with 8 to 22 carbon atoms in the alkyl residue and the ethoxylated analogues thereof,
- ethoxylated sterols,
- partial esters of polyglycerols with n = 2 to 10 glycerol units and esterified with 1 to 4 saturated or unsaturated, linear or branched, optionally hydroxylated C₈-C₃₀ fatty acid residues, provided that they exhibit an HLB value of more than 7,
- and mixtures of the above-stated substances.

6. A deodorant or antiperspirant stick according to one of the preceding claims, **characterised in that** the oil-in-water emulsifier b) is present in a total quantity of 1-4 wt.%, relative to the total composition.

7. A deodorant or antiperspirant stick according to one of the preceding claims, **characterised in that** the water-in-oil emulsifier c) is present in a total quantity of 0.5-8 wt.%, and preferably of 1-4 wt.%, relative to the total weight of the composition.

8. A deodorant or antiperspirant stick according to one of the preceding claims, **characterised in that** the oil d) which is liquid at 20°C is selected from
- branched saturated or unsaturated fatty alcohols with 6-30 carbon atoms,
- triglycerides of linear or branched, saturated or unsaturated, optionally hydroxylated C₈₋₃₀ fatty acids,
- dicarboxylic acid esters of linear or branched C₂-C₁₀ alkanols,
- esters of branched saturated or unsaturated fatty alcohols with 2-30 carbon atoms with linear or branched saturated or unsaturated fatty acids with 2-30 carbon atoms, which may be hydroxylated,
- addition products of 1 to 5 propylene oxide units onto mono- or polyhydric C₈₋₂₂ alkanols,
- addition products of at least 6 ethylene oxide and/or propylene oxide units onto mono- or polyhydric C₃₋₂₂ alkanols,
- C₈-C₂₂ fatty alcohol esters of monobasic or polybasic C₂-C₇ hydroxycarboxylic acids,
- symmetrical, asymmetrical or cyclic esters of carbonic acid with fatty alcohols,
- the esters of dimers of unsaturated C₁₂-C₂₂ fatty acids (dimer fatty acids) with monohydric linear, branched or cyclic C₂-C₁₈ alkanols or with polyhydric linear or branched C₂-C₆ alkanols,
- and mixtures of the above-stated substances.

9. A deodorant or antiperspirant stick according to one of the preceding claims, **characterised in that** the oil d) which is liquid at 20°C is present in a total quantity of 5-14 wt.%, relative to the total weight of the composition.

10. A deodorant or antiperspirant stick according to one of the preceding claims, **characterised in that** the oils d) which are liquid at 20°C contain at most 20 wt.% of oil(s), relative to the total weight of oils which are liquid at 20°C, the solubility parameter of which deviates by more than -1.67 (J/cm³)^{0.5} or -2.93 (J/cm³)^{0.5} (-0.4 or -0.7 (cal/cm³)^{0.5}) or by more than +2.93 (J/cm³)^{0.5} (+0.7 (cal/cm³)^{0.5}) from the (average) solubility parameter of the water-in-oil emulsifier(s) c).

11. A deodorant or antiperspirant stick according to one of the preceding claims, **characterised in that** no oils which are liquid at 20°C are present, the solubility parameter of which deviates by more than ±4.1868 (J/cm³)^{0.5} (±1.0 (cal/cm³)^{0.5}) from the (average) solubility parameter of the water-in-oil emulsifier(s) c).

12. A deodorant or antiperspirant stick according to one of the preceding claims, **characterised in that** the water-soluble polyhydric C₂-C₉ alkanol with 2-6 hydroxyl groups and/or the water-soluble polyethylene glycol with 3-20 ethylene oxide units is present in total in quantities of 8-18 wt.%, relative to the total composition.

13. A deodorant or antiperspirant stick according to one of the preceding claims, **characterised in that** additionally least one lipid or wax component with a melting point in the range from 25-<50°C, selected from coconut oil fatty acid glycerol mono-, di- and triesters, *Butyrospermum parkii* (shea butter) and esters of saturated, monohydric C₈-C₁₈ alcohols with saturated C₁₂-C₁₈ monocarboxylic acids and mixtures of these substances, is present.

14. A deodorant or antiperspirant stick according to claim 13, **characterised in that** the at least one lipid or wax component with a melting point in the range from 25-<50°C is present in quantities of 0.01 to 20 wt.%, preferably of 3-20 wt.%, particularly preferably of 5-18 wt.% and extremely preferably of 6-15 wt.%, relative to the total composition.

15. A deodorant or antiperspirant stick according to one of the preceding claims, **characterised in that** 10 to less than 30 wt.% of water, relative to the total composition, is present.

## Revendications

1. Déodorant ou antitranspirant en bâton sous la forme d'une dispersion de type huile-dans-l'eau, contenant :
a) au total à concurrence de 4 à 20 % en poids, au moins un composant de lipide ou de cire possédant un point de fusion supérieure à 50 °C, qui ne doit pas être classé dans le composant b) ou c) ;
b) un mélange d'émulsifiant non ionique de type huile-dans-l'eau possédant une valeur HLB moyenne dans la plage de 10 à 19, contenant, rapportés à la composition totale, à concurrence de 0,5 à 10 % en poids, au moins un émulsifiant non ionique de type huile-dans-l'eau possédant une valeur HLB supérieure à 7 ;
c) au total à concurrence de 0,1 à 15 % en poids, au moins un émulsifiant non ionique de type eau-dans-l'huile possédant une valeur HLB supérieure à 1,0 et inférieure ou égale à 7,0, qui peut former, avec de l'eau uniquement ou bien avec de l'eau en présence d'un émulsifiant hydrophile, des structures de cristaux liquides, et qui est choisi parmi :
- des alcanols linéaires saturés en C₁₂-C₃₀ ;
- des monoesters et des diesters d'éthylèneglycol d'acides carboxyliques en C₁₂-C₃₀ linéaires, saturés et insaturés, qui peuvent être hydroxylés ;
- des monoesters et des diesters de glycérol d'acides carboxyliques en C₁₂-C₃₀ linéaires, saturés et insaturés, qui peuvent être hydroxylés ;
- des monoesters et des diesters de propylèneglycol d'acides carboxyliques en C₁₂-C₃₀ linéaires, saturés et insaturés, qui peuvent être hydroxylés ;
- des monoesters, des diesters et des triesters de sorbitanne d'acides carboxyliques en C₁₂-C₃₀ linéaires, saturés et insaturés, qui peuvent être hydroxylés ;
- des monoesters, des diesters, des triesters et des tétraesters de pentaérythrityle d'acides carboxyliques en C₁₂-C₃₀ linéaires, saturés et insaturés, qui peuvent être hydroxylés ;
- des monoesters et des diesters de méthylglucose d'acides carboxyliques en C₁₂-C₃₀ linéaires, saturés et insaturés, qui peuvent être hydroxylés ;
- des stérols ;
- des alcanols et des acides carboxyliques comprenant respectivement de 8 à 24 atomes de carbone, en particulier de 16 à 22 atomes de carbone, dans le groupe alkyle, et de 1 à 4 unités d'oxyde d'éthylène par molécule, qui présentent une valeur HLB supérieure à 1,0 et inférieure ou égale à 7,0 ;
- des monoéthers de glycérol d'alcools saturés et/ou insaturés, ramifiés et/ou non ramifiés, possédant une longueur de chaîne de 8 à 30 atomes de carbone, en particulier de 12 à 18 atomes de carbone ;
- des esters partiels de polyglycérols, dans lesquels n représente de 2 à 10 unités de glycérol et qui sont estérifiés avec de 1 à 5 esters d'acides gras en C₈-C₃₀ saturés ou insaturés, linéaires ou ramifiés, le cas échéant hydroxylés, pour autant qu'ils présentent une valeur HLB inférieure ou égal à 7 ;
- ainsi que des mélanges des substances susmentionnées ; à titre d'agent procurant une consistance et/ou à titre d'agent liant l'eau ;
d) au total à concurrence de 3 à 20 % en poids, au moins une huile liquide à 20 °C, qui ne représente ni un composant faisant office d'arôme, ni une huile éthérée, le paramètre de solubilité (moyenne) de la totalité des huiles d) contenues dans la dispersion, en présence d'alcools gras linéaires saturés possédant une longueur de chaîne d'au moins 8 atomes de carbone, s'écartant au maximum à concurrence de -2,93 (J/cm³)^{0,5} (-0,7 (cal/cm³)^{0,5}, resp. au maximum à concurrence de +2,93 (J/cm³)^{0,5} (+0,7 (cal/cm³)^{0,5}, et en présence d'émulsifiants du type eau-dans-l'huile qui sont différents d'alcools gras saturés et linéaires possédant une longueur de chaîne d'au moins 8 atomes de carbone en l'absence d'alcools gras saturés linéaires possédant une longueur de chaîne d'au moins 8 atomes de carbone, s'écartant au maximum à concurrence de -1,67 (J/cm³)^{0,5} (-0,4 (cal/cm³)^{0,5}, resp. au maximum à concurrence de +2,93 (J/cm³)^{0,5} (+0,7 (cal/cm³)^{0,5} du paramètre de solubilité (moyenne) de l'émulsifiant du type eau-dans-l'huile/des émulsifiants du type eau-dans-l'huile;
e) au total à concurrence de 3 à 25 % en poids, au moins un alcanol en C₂-C₉ polyvalent, soluble dans l'eau, comprenant de 2 à 6 groupes hydroxyle et/ou au moins un polyéthylèneglycol soluble dans l'eau comprenant de 3 à 20 unités d'oxyde d'éthylène ;
f) à concurrence de 10 à moins de 50 % en poids, de l'eau, rapportés à la composition totale ;
g) au moins une substance active déodorante ou antitranspirante ; le bâton présentant
h) une valeur de force de pénétration dans la plage de 150 à 800 gramme-force (g-force) à une profondeur de pénétration de 5000 mm; et
i) une résistance électrique maximale de 300 kΩ (kilo-ohm), que l'on mesure conformément au procédé révélé dans la description ; et
j) la solidification du déodorant ou de l'antitranspirant sous forme de bâton n'ayant lieu ni sur base de gels de savons respectivement de gels de sels d'acides gras, ni sur base de formateurs d'hydrogels polymères inorganiques et/ou organiques, le terme « acide gras » désignant des acides alcanoïques, alcénoiques et alcynoiques comprenant au moins 4 atomes de carbone ;
le paramètre de solubilité étant déterminé conformément à un procédé selon lequel l'adipate de diisopropyle présente un paramètre de solubilité de 35,42 (J/cm³)^{0,5} (8,46 (cal/cm³)^{0,5}) et l'octyldodécanol présente un paramètre de solubilité de 37,35 (J/cm³)^{0,5} (8,92 (cal/cm³)^{0,5}) , toutes les proportions se rapportant à la composition totale.

2. Déodorant ou antitranspirant en bâton selon la revendication 1, **caractérisé en ce que** le composant de lipide ou de cire a) est choisi parmi des esters d'un alcanol monovalent saturé en C₁₆-C₆₀ et d'un acide monocarboxylique saturé en C₈-C₃₆, en particulier le béhénate de cétyle, le béhénate de stéaryle et le stéarate d'alkyle en C₂₀-C₄₀, des triesters de glycérol d'acides carboxyliques linéaires saturés en C₁₂-C₃₀ qui peuvent être hydroxylés, de la cire de candélilla, de la cire de carnauba, de la cire d'abeilles, des acides carboxyliques linéaires saturés en C₁₄-C₃₆, ainsi que des mélanges des substances susmentionnées.

3. Déodorant ou antitranspirant en bâton selon l'une quelconque des revendications précédentes, **caractérisé en ce que** le composant de lipide ou de cire a) est contenu au total dans des quantités de 8 à 15 % en poids, rapportés à la composition totale.

4. Déodorant ou antitranspirant en bâton selon la revendication 2, **caractérisé en ce que** l'ester/les esters d'un alcanol monovalent saturé en C₁₆-C₆₀ et d'un acide monocarboxylique saturé en C₈-C₃₆ est/sont contenu(s) dans des quantités de 2 à 10 % en poids, de préférence de 2 à 6 % en poids, rapportés à la composition totale.

5. Déodorant ou antitranspirant en bâton selon l'une quelconque des revendications précédentes, **caractérisé en ce que** l'émulsifiant non ionique de type huile-dans-l'eau b) possédant une valeur HLB supérieure à 7 est choisi parmi :
- des alcanols éthoxylés en C₈-C₂₄ possédant en moyenne de 10 à 100 mol d'oxyde d'éthylène par mole ;
- des acides carboxyliques éthoxylés en C₈-C₂₄ possédant en moyenne de 10 à 100 mol d'oxyde d'éthylène par mole ;
- des copolyols de silicone-polyols comprenant des unités d'oxyde d'éthylène ou des unités d'oxyde d'éthylène et d'oxyde de propylène ;
- des alkyl-mono- et oligo-glycosides contenant de 8 à 22 atomes de carbone dans le radical alkyle, ainsi que leurs analogues éthoxylés ;
- des stérols éthoxylés ;
- des esters partiels de polyglycérol dans lesquels n représente de 2 à 10 unités de glycérol et qui sont estérifiés avec de 1 à 4 esters d'acides gras en C₈-C₃₀ saturés ou insaturés, linéaires ou ramifiés, le cas échéant hydroxylés, pour autant qu'ils présentent une valeur HLB supérieure à 7 ;
- ainsi que des mélanges des substances susmentionnées.

6. Déodorant ou antitranspirant en bâton selon l'une quelconque des revendications précédentes, **caractérisé en ce que** l'émulsifiant b) du type huile-dans-l'eau est contenu en une quantité totale de 1 à 4 % en poids, rapportés à la composition totale.

7. Déodorant ou antitranspirant en bâton selon l'une quelconque des revendications précédentes, **caractérisé en ce que** l'émulsifiant c) du type eau-dans-l'huile est contenu en une quantité totale de 0,5 à 8 % en poids, et de préférence de 1 à 4 % en poids, rapportés au poids total de la composition.

8. Déodorant ou antitranspirant en bâton selon l'une quelconque des revendications précédentes, **caractérisé en ce que** l'huile liquide à 20 °C d) est choisie parmi :
- des alcools gras ramifiés, saturés ou insaturés, contenant de 6 à 30 atomes de carbone ;
- des triglycérides d'acides gras en C₈-C₃₀ linéaires ou ramifiés, saturés ou insaturés, le cas échéant hydroxylés ;
- des esters d'acides dicarboxyliques d'alcanols linéaires ou ramifiés en C₂-C₁₀ ;
- des esters d'alcools gras ramifiés, saturés ou insaturés contenant de 2 à 30 atomes de carbone avec des acides gras linéaires ou ramifiés, saturés ou insaturés contenant de 2 à 30 atomes de carbone, qui peuvent être hydroxylés ;
- des produits de fixation par addition de 1 à 5 unités d'oxyde de propylène sur des alcanols monovalents ou polyvalents en C₈-C₂₂ ;
- des produits de fixation par addition d'au moins 6 unités d'oxyde d'éthylène et/ou de propylène sur des alcanols monovalents ou polyvalents en C₃-C₂₂ ;
- des esters d'alcools gras en C₈-C₂₂ d'acides hydroxycarboxyliques monovalents ou polyvalents en C₂-C₇;
- des esters symétriques, asymétriques ou cycliques de l'acide carbonique avec des alcools gras ;
- des esters de dimères d'acides gras insaturés en C₁₂-C₂₂ (acides gras dimères) avec des alcanols monovalents, linéaires, ramifiés ou cycliques en C₂-C₁₈ ou avec des alcanols polyvalents, linéaires ou ramifiés en C₂-C₆ ;
- ainsi que des mélanges des substances susmentionnées.

9. Déodorant ou antitranspirant en bâton selon l'une quelconque des revendications précédentes, **caractérisé en ce que** l'huile liquide à 20 °C d) est contenue en une quantité totale de 5 à 14 % en poids, rapportés au poids total de la composition.

10. Déodorant ou antitranspirant en bâton selon l'une quelconque des revendications précédentes, **caractérisé en ce que** les huiles liquides à 20 °C d) contiennent, à concurrence maximale de 20 % en poids, rapportés au poids total des huiles liquides à 20 °C, une huile ou des huiles dont le paramètre de solubilité s'écarte à concurrence d'une valeur supérieure à -1,67 (J/cm³)^{0,5}, resp., à -2,93 (J/cm³)^{0,5} (-0,4, resp., -0,7 (cal/CM³)^{0,5} ou à concurrence d'une valeur supérieure à +2,93 (J/cm³)^{0,5} (+0,7 (cal/cm³)^{0,5} du paramètre de solubilité (moyenne) de l'émulsifiant du type eau-dans-l'huile/des émulsifiants du type eau-dans-l'huile c).

11. Déodorant ou antitranspirant en bâton selon l'une quelconque des revendications précédentes, **caractérisé en ce qu'**il ne contient aucune huile liquide à 20 °C dont le paramètre de solubilité s'écarte à concurrence d'une valeur supérieure à ± 4,1868 (J/cm³)^{0,5} (± 1,0 (cal/cm³)^{0,5} du paramètre de solubilité (moyenne) de l'émulsifiant du type eau-dans-l'huile/des émulsifiants du type eau-dans-l'huile c).

12. Déodorant ou antitranspirant en bâton selon l'une quelconque des revendications précédentes, **caractérisé en ce que** l'alcanol en C₂-C₉ polyvalent, soluble dans l'eau contenant de 2 à 6 groupes hydroxyle et/ou le polyéthylèneglycol soluble dans l'eau contenant de 3 à 20 unités d'oxyde d'éthylène sont contenus au total dans des quantités de 8 à 18 % en poids, rapportés à la composition totale.

13. Déodorant ou antitranspirant en bâton selon l'une quelconque des revendications précédentes, **caractérisé en ce que** qu'il contient en outre au moins un composant de lipide ou de cire possédant un point de fusion dans la plage de 25 à une valeur inférieure à 50 °C, choisi parmi des monoesters, des diesters et des triesters de glycérol d'acides gras de coco, Butyrospernum Parkii (beurre de karité) et des esters d'alcools gras, monovalents en C₈-C₁₈ avec des acides monocarboxyliques saturés en C₁₂-C₁₈, et des mélanges de ces substances.

14. Déodorant ou antitranspirant en bâton selon la revendication 13, **caractérisé en ce que** le composant de lipide ou de cire possédant un point de fusion dans la plage de 25 à une valeur inférieure à 50 °C est contenu dans des quantités de 0,01 à 20 % en poids, de préférence dans des quantités de 3 à 20 % en poids, de manière particulièrement préférée dans des quantités de 5 à 18 % en poids et de manière extraordinairement préférée dans des quantités de 6 à 15 % en poids, rapportés à la composition totale.

15. Déodorant ou antitranspirant en bâton selon l'une quelconque des revendications précédentes, **caractérisé en ce qu'**il contient de l'eau à concurrence de 10 à une valeur inférieure à 30 % en poids, rapportés à la composition totale.
